# EUROPEAN PATENT APPLICATION

(11) **EP 4 190 317 A1**
(43) Date of publication of application: **07.06.2023**
(21) Application number: 21864654.5
(22) Date of filing: 01.09.2021
(51) Int. Cl.: A61K 9/70, A61K 47/10, A61K 47/36, A61K 47/42, A61P 21/00, A61P 17/02, A61P 41/00, A61L 27/52, A61L 27/54

(54) **EXTRACELLULAR MATRIX-SUPPORTED BIOMIMETIC TISSUE ADHESIVE HYDROGEL PATCH**

(30) Priority: 01.09.2020 KR 20200111336
(71) Applicant: Cellartgen Inc., Seoul 03722 (KR)
(72) Inventor: CHO, Seung Woo, Seoul 03722 (KR); JEON, Eun Je, Seoul 03722 (KR)
(74) Representative: Manitz Finsterwald Patent- und Rechtsanwaltspartnerschaft mbB
(86) International application number: PCT/KR2021/011782
(87) International publication number: WO 2022/050695

(57) **Abstract**

The present disclosure relates to a hydrogel patch, including: a hydrogel patch containing a biocompatible polymer functionalized with a phenol group; and an extracellular matrix loaded in the hydrogel patch.

## Description

### TECHNICAL FIELD

The present disclosure relates to an extracellular matrix-loaded bio-inspired tissue-adhesive hydrogel patch.

### BACKGROUND

The global biomaterials market was valued at $105 billion in 2019 and is expected to reach $207 billion by 2024 at a continuous compound annual growth rate of 14.5%. The demand for biomaterials in various medical fields, such as engraftable materials and materials for plastic surgery and wound healing, is rapidly increasing. In particular, as South Korea becomes a super-aged society, the demand for functional biomaterials that can substitute for real tissues in vivo is on the rise due to physical damage caused by various diseases and aging.

Meanwhile, volumetric muscle loss caused by accidents, surgery, etc. and sarcopenia caused by diseases and aging are diseases that must be treated because they affect not only the muscle itself but also the entire body including bones, blood vessels, nerves, liver, heart and pancreas. In particular, sarcopenia caused by aging has not been classified as a disease until recently, but has been managed after being designated a disease code in the United States and Japan in 2016 and 2018, respectively. Thus, sarcopenia is recognized as a disease that requires the development of a therapeutic agent.

The global anti-aging market, including senile sarcopenia, was valued at $62.5 billion in 2017 and is expected to expand to $88.6 billion (109 trillion won) by 2022 at a continuous compound annual growth rate of 6.5%. Currently, the US and Europe are leading the market for anti-aging products and pharmaceuticals, but the Asian market, such as China and India, including Korea and Japan which already entered a super-aged society is expected to lead the future growth and also expected to grow further.

Tissue-derived extracellular matrix components are composed of materials, such as glycoproteins and proteoglycans, that can induce various tissue-specific physiological activities, and are being actively researched as therapeutic agents for disease treatment and tissue regeneration. The tissue-derived extracellular matrix components are functional biomaterials that have recently been applied clinically and commercialized. However, according to most of the prior art, the extracellular matrix components are delivered into the body by injecting them like a drug or injecting them prepared in the form of a hydrogel. However, according to the prior art, it is difficult to effectively deliver the extracellular matrix components into the body and to maintain its efficacy in vivo for a long time.

Therefore, in order to overcome the limitations of the prior art, the present disclosure developed a functional hydrogel patch system which is fabricated by fusing a hyaluronic acid derivative functionalized with a phenol group and a muscle tissue-specific extracellular matrix to have excellent biocompatibility, is very easy to control in terms of physical properties depending on the use, and can mimic a tissue-specific microenvironment. The muscle tissue-specific extracellular matrix component loaded in the phenol group-functionalized hyaluronic acid patch is a therapeutic material that promotes the proliferation and differentiation of muscle stem cells through the realization of a muscle microenvironment, and can be applied to the treatment of muscle loss and myopathy.

In addition, various studies on stem cells and gene therapy are being conducted to treat volumetric muscle loss (VML) and degenerative muscle diseases (muscular dystrophy and sarcopenia) that exceed the self-renewal ability of muscle. However, there is still no fundamental treatment method. As for volumetric muscle loss, any proper tissue reconstruction method has not been developed clinically except for surgical autologous muscle tissue graft. Also, muscular dystrophy and sarcopenia are caused by various factors, such as aging, genetic variation, metabolic disorder, etc., and, thus, any proper treatment method other than exercise prescription, physical therapy, drug treatment and diet has not been developed. Even these are insufficient to maintain adequate muscle mass and suppress degenerative muscle loss for a long time.

Therefore, in order to solve these medical difficulties, it is necessary to develop biomaterials and drug delivery methods that can promote the proliferation and differentiation of muscle stem cells present in the human body. The phenol group-functionalized hyaluronic acid hydrogel patch cross-linked by using the muscle extracellular matrix developed in the present disclosure can be easily attached to a desired site without the use of additional oxidizing agents or medical adhesives, and provides efficient delivery of muscle extracellular matrix components and thus activates a small amount of muscle stem cells present in damaged muscle tissue. Therefore, it can greatly enhance the muscle tissue regeneration activity through cell proliferation and differentiation, showing the potential as a new medical technology for fundamental muscle tissue reconstruction.

### DISCLOSURE OF THE INVENTION

### PROBLEMS TO BE SOLVED BY THE INVENTION

The present disclosure is intended to fabricate an extracellular matrix-loaded bio-inspired tissue-adhesive hydrogel patch with excellent mechanical properties, tissue adhesion, biocompatibility and ease of use, and provides an extracellular matrix hydrogel patch, including: a hydrogel patch containing a biocompatible polymer functionalized with a phenol group; and an extracellular matrix loaded in the hydrogel patch.
means for solving the problems

An aspect of the present disclosure provides an extracellular matrix hydrogel patch, including: a hydrogel patch containing a biocompatible polymer functionalized with a phenol group; and an extracellular matrix loaded in the hydrogel patch.

In an embodiment of the present disclosure, the phenol group may be a catechol group derived from a catechol-based compound selected from the group consisting of catechol, 4-tert-butylcatechol (TBC), urushiol, alizarin, dopamine, dopamine hydrochloride, 3,4-dihydroxyphenylalanine (DOPA), caffeic acid, norepinephrine, epinephrine, 3,4-dihydroxyphenylacetic acid (DOPAC), isoprenaline, isoproterenol and 3,4-dihydroxybenzoic acid; or a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

In an embodiment of the present disclosure, the biocompatible polymer may be selected from the group consisting of hyaluronic acid, heparin, cellulose, dextran, alginate, chitosan, chitin, collagen, gelatin, chondroitin sulfate, pectin, keratin and fibrin.

In an embodiment of the present disclosure, the extracellular matrix hydrogel patch may have i) a thickness of from 0.05 mm to 10.0 mm, ii) a storage modulus G' of from 1×10² Pa to 1×10⁶ Pa and tanδ of from 0.2 to 0.5 in a frequency range of from 0.1 Hz to 10 Hz, iii) a friction factor of from 0.2 to 0.4 as measured at a speed of 0.01 m/s under a normal force of 5 N, and iv) an adhesive strength of from 1 N to 10 N.

In an embodiment of the present disclosure, the extracellular matrix may serve as a cross-linking agent via interaction with the phenol group.

In an embodiment of the present disclosure, the extracellular matrix may be derived from any one or more tissues selected from the group consisting of muscle, brain, spinal cord, tongue, airway, skin, lymph, lung, heart, liver, stomach, kidney, spleen, pancreas, intestine, adrenal gland, fat, uterus, thymus, esophagus, salivary gland, bone, bladder, blood vessel, tendon, thyroid and gum.

### Effects of the invention

In order to overcome the limitations of the conventional muscle disease treatment and muscle regeneration technology, the inventors of the present disclosure succeeded in developing a new paradigm for therapeutic agent delivery system that applies a muscle tissue extracellular matrix as a therapeutic agent and a component for the production of a hydrogel biomaterial. The extracellular matrix delivery technology developed in the present disclosure is a freeze-dried hydrogel patch formulation, and, thus, it is possible to maintain the activity of the therapeutic agent for a long time. Also, it can be stored for a long time and is easy to use, and, thus, it has a very high potential for practical use. Therefore, it is considered as a new technology for treating muscle loss and sarcopenia for which any suitable therapeutic agent is available till date and expected to create economic benefits and high added value through commercialization. This technology makes it possible to deliver extracellular matrices derived from various tissues, and, thus, the scope of applicable diseases can be expanded. Accordingly, it can be developed as a therapeutic agent for various intractable diseases as well as muscle diseases.

### BRIEF DESCRIPTION OF THE DRAWINGS

**FIG. 1A** shows the structural formula of HA-CA.
**FIG. 1B** shows the result of analyzing a swelling ratio of an HA-CA hydrogel patch.
**FIG. 1C** shows the result of analyzing a degradation rate of the HA-CA hydrogel patch by an enzyme.
**FIG. 2A** shows the result of analyzing a storage modulus and a loss modulus of the HA-CA hydrogel patch.
**FIG. 2B** shows the result of analyzing an average storage modulus of the HA-CA hydrogel patch.
**FIG. 3A** shows the result of analyzing a friction factor of the HA-CA hydrogel patch.
**FIG. 3B** shows the result of analyzing the degree of wear scar of the HA-CA hydrogel patch.
**FIG. 3C** shows the result of analyzing the area of wear scar of the HA-CA hydrogel patch.
**FIG. 4A** shows the structural formula of HA-PG.
**FIG. 4B** shows the result of analyzing a swelling ratio of an HA-PG hydrogel patch.
**FIG. 4C** shows the result of analyzing a degradation rate of the HA-PG hydrogel patch by an enzyme.
**FIG. 5A** shows the result of analyzing a storage modulus and a loss modulus of the HA-PG hydrogel patch.
**FIG. 5B** shows the result of analyzing an average storage modulus of the HA-PG hydrogel patch.
**FIG. 6A** shows the result of analyzing a friction factor of the HA-PG hydrogel patch.
**FIG. 6B** shows the result of analyzing the area of wear scar of the HA-PG hydrogel patch.
**FIG. 6C** shows the result of analyzing the degree of wear scar of the HA-PG hydrogel patch.
**FIG. 7** shows a process of fabricating an HA-CA hydrogel patch for delivery of a muscle extracellular matrix (MEM).
**FIG. 8a** shows a method of fabricating an MEM used for preparing an MEM hydrogel patch and the result of analysis.
**FIG. 8b** shows a method of fabricating an MEM used for preparing an MEM hydrogel patch and the result of analysis.
**FIG. 8c** shows a method of fabricating an MEM used for preparing an MEM hydrogel patch and the result of analysis.
**FIG. 8d** shows a method of fabricating an MEM used for preparing an MEM hydrogel patch and the result of analysis.
**FIG. 9a** shows the result of proteomic analysis of the fabricated MEM.
**FIG. 9b** shows the result of proteomic analysis of the fabricated MEM.
**FIG. 9c** shows the result of proteomic analysis of the fabricated MEM.
**FIG. 9d** shows the result of proteomic analysis of the fabricated MEM.
**FIG. 10a** shows the result of analyzing a chemical cross-linking mechanism of an MEM/HA-CA hydrogel patch.
**FIG. 10b** shows the result of analyzing a chemical cross-linking mechanism of an MEM/HA-CA hydrogel patch.
**FIG. 11a** shows the result of analyzing the mechanical properties and adhesive properties of the MEM/HA-CA hydrogel patch.
**FIG. 11b** shows the result of analyzing the mechanical properties and adhesive properties of the MEM/HA-CA hydrogel patch.
**FIG. 11c** shows the result of analyzing the mechanical properties and adhesive properties of the MEM/HA-CA hydrogel patch.
**FIG. 11d** shows the result of analyzing the mechanical properties and adhesive properties of the MEM/HA-CA hydrogel patch.
**FIG. 12a** shows the result of comparison in mechanical properties depending on the cross-linking method of an MEM/HA-CA hydrogel.
**FIG. 12b** shows the result of comparison in mechanical properties depending on the cross-linking method of an MEM/HA-CA hydrogel.
**FIG. 13a** shows the result of analyzing the physical properties of the MEM/HA-CA hydrogel patch by identifying swelling and degradation patterns.
**FIG. 13b** shows the result of analyzing the physical properties of the MEM/HA-CA hydrogel patch by identifying swelling and degradation patterns.
**FIG. 14a** shows the result of checking the possibility of sustained MEM delivery by the MEM/HA-CA hydrogel patch.
**FIG. 14b** shows the result of checking the possibility of sustained MEM delivery by the MEM/HA-CA hydrogel patch.
**FIG. 15a** shows the result of checking the biocompatibility of the MEM/HA-CA hydrogel patch.
**FIG. 15b** shows the result of checking the biocompatibility of the MEM/HA-CA hydrogel patch.
**FIG. 15c** shows the result of checking the biocompatibility of the MEM/HA-CA hydrogel patch.
**FIG. 16a** shows the result of checking the effect of enhancing muscle stem cell activation by the MEM/HA-CA hydrogel patch.
**FIG. 16b** shows the result of checking the effect of enhancing muscle stem cell activation by the MEM/HA-CA hydrogel patch.
**FIG. 16c** shows the result of checking the effect of enhancing muscle stem cell activation by the MEM/HA-CA hydrogel patch.
**FIG. 16d** shows the result of checking the effect of enhancing muscle stem cell activation by the MEM/HA-CA hydrogel patch.
**FIG. 16e** shows the result of checking the effect of enhancing muscle stem cell activation by the MEM/HA-CA hydrogel patch.
**FIG. 16f** shows the result of checking the effect of enhancing muscle stem cell activation by the MEM/HA-CA hydrogel patch.
**FIG. 17a** shows the result of checking the application of the MEM/HA-CA hydrogel patch onto a volumetric muscle loss model and the effect of regenerating muscle tissue.
**FIG. 17b** shows the result of checking the application of the MEM/HA-CA hydrogel patch onto a volumetric muscle loss model and the effect of regenerating muscle tissue.
**FIG. 17c** shows the result of checking the application of the MEM/HA-CA hydrogel patch onto a volumetric muscle loss model and the effect of regenerating muscle tissue.
**FIG. 18a** shows the result of checking the effect of improving muscle function by the MEM/HA-CA hydrogel patch on the volumetric muscle loss model.
**FIG. 18b** shows the result of checking the effect of improving muscle function by the MEM/HA-CA hydrogel patch on the volumetric muscle loss model.
**FIG. 19a** shows the result of checking the effect of tissue regeneration by the MEM/HA-CA hydrogel patch on the volumetric muscle loss model.
**FIG. 19b** shows the result of checking the effect of tissue regeneration by the MEM/HA-CA hydrogel patch on the volumetric muscle loss model.
**FIG. 19c** shows the result of checking the effect of tissue regeneration by the MEM/HA-CA hydrogel patch on the volumetric muscle loss model.
**FIG. 19d** shows the result of checking the effect of tissue regeneration by the MEM/HA-CA hydrogel patch on the volumetric muscle loss model.
**FIG. 19e** shows the result of checking the effect of tissue regeneration by the MEM/HA-CA hydrogel patch on the volumetric muscle loss model.
**FIG. 19f** shows the result of checking the effect of tissue regeneration by the MEM/HA-CA hydrogel patch on the volumetric muscle loss model.
**FIG. 19g** shows the result of checking the effect of tissue regeneration by the MEM/HA-CA hydrogel patch on the volumetric muscle loss model.
**FIG. 19h** shows the result of checking the effect of tissue regeneration by the MEM/HA-CA hydrogel patch on the volumetric muscle loss model.
**FIG. 19i** shows the result of checking the effect of tissue regeneration by the MEM/HA-CA hydrogel patch on the volumetric muscle loss model.
**FIG. 20** shows a schematic diagram showing the development of a phenol group (gallol group)-functionalized hydrogel patch (MEM/HA-PG) for delivery of a muscle extracellular matrix (MEM).
**FIG. 21** shows the result of analyzing the mechanical properties of the MEM/HA-PG hydrogel patch.
**FIG. 22a** shows the result of checking the biocompatibility of the MEM/HA-PG hydrogel patch.
**FIG. 22b** shows the result of checking the biocompatibility of the MEM/HA-PG hydrogel patch.
**FIG. 22c** shows the result of checking the biocompatibility of the MEM/HA-PG hydrogel patch.
**FIG. 22d** shows the result of checking the biocompatibility of the MEM/HA-PG hydrogel patch.

### BEST MODE FOR CARRYING OUT THE INVENTION

The present inventors have developed a technology for delivering a tissue-derived extracellular matrix into the body using a phenol group (catechol group, gallol group)-functionalized hyaluronic acid hydrogel patch that mimics the adhesive components of mussels and sea squirts.

Specifically, in order to perform cross-linking of a hydrogel functionalized with a phenol group, a cross-linking agent such as an oxidizing agent needs to be added. However, in the present disclosure, a hydrogel was successfully obtained by applying a tissue-derived extracellular matrix component to induce cross-linking without the use of a cross-linking agent. As for the hyaluronic acid functionalized with a phenol group, cross-linking occurs via reaction between phenol groups oxidized after treatment with the cross-linking agent. In the present disclosure, a muscle extracellular matrix was added based on the fact that a phenol group has high reactivity with functional groups contained in proteins and peptides, and, thus, cross-linking was induced via reactions with various functional groups present in the extracellular matrix component without treatment of an oxidizing agent. Further, the physical, chemical and mechanical properties of the hydrogel were controlled by modulating the concentration of the extracellular matrix component. That is, it was confirmed that the extracellular matrix component can serve as both a therapeutic material to be delivered for tissue regeneration and a cross-linking agent.

A cross-linking mechanism of the hydrogel functionalized with a phenol group by the tissue-derived extracellular matrix component was identified through chemical analysis. It was confirmed that when a muscle extracellular matrix is added to a hyaluronic acid derivative functionalized with a phenol group, the number of non-covalent bonds (e.g., hydrogen bonds) between the two materials is greatly increased and the formation of covalent bonds between catechols (e.g., formation of dicatechol) is promoted, which results in the cross-linking effect. Also, it was confirmed that amide bonds introduced from a large amount of protein enhance the stability of the three-dimensional hydrogel. As such, by developing a new cross-linking method applying a therapeutic material, which is a target to be delivered into the body, the problem of using a cross-linking agent (oxidizing agent), which has been the biggest obstacle to the clinical application of conventional hydrogels functionalized with a phenol group, can be solved and a drug delivery system with improved safety is developed.

The tissue-derived extracellular matrix added for cross-linking is produced through decellularization that can remove cells. Thus, when the tissue-derived extracellular matrix is applied in vivo, it can minimize immune responses. The extracellular matrix prepared as such mainly form non-covalent bonds with the hyaluronic acid functionalized with a phenol group to form a hydrogel. Therefore, various tissue-specific glycoproteins, proteoglycans and active materials involved in cross-linking can induce their intrinsic physiological activities without loss of functionality. By using these characteristics, a new drug delivery system that can efficiently deliver a therapeutic material for treating specific tissue damage and inducing tissue regeneration has been successfully developed.

In the present disclosure, it was confirmed that various properties such as swelling ratio, degradation rate, mechanical properties and adhesive properties of the hyaluronic acid hydrogel functionalized with a phenol group can be easily controlled by regulating the concentration of the muscle extracellular matrix. Also, it was confirmed that muscle stem cells as satellite cells are activated to induce cell proliferation and contribute to muscle tissue regeneration. Further, when the developed hydrogel patch system for extracellular matrix delivery was applied to a muscle-damaged animal model, the hydrogel patch was stably attached to a muscle defect site without a separate adhesive due to its excellent tissue adhesive properties and the exercise capacity of the animal model was greatly improved. That is, it was able to induce the effect of structural and functional regeneration of muscle tissue.

The present disclosure provides an extracellular matrix hydrogel patch, including: a hydrogel patch containing a biocompatible polymer functionalized with a phenol group; and an extracellular matrix loaded in the hydrogel patch.

First, the hydrogel patch according to the present disclosure includes a hydrogel patch containing a biocompatible polymer functionalized with a phenol group.

As used herein, the term "phenol group" is a functional group derived from a phenol-based compound, desirably a functional group derived from a catechol-based compound including 1,2-dihydroxybenzene having two hydroxyl groups (-OH) located adjacent to each other or a functional group derived from a pyrogallol-based compound including 1,2,3-trihydroxybenzene having three hydroxyl groups (-OH) located adjacent to each other, at the terminal. The phenol group can form covalent cross-linking with various functional groups through an oxidation reaction. Desirably, the hydrogel patch may further include a terminal functional group for reaction with the biocompatible polymer in addition to the phenol group, but is not limited thereto.

Specifically, the catechol-based compound may be selected from the group consisting of catechol, 4-tert-butylcatechol (TBC), urushiol, alizarin, dopamine, dopamine hydrochloride, 3,4-dihydroxyphenylalanine (DOPA), caffeic acid, norepinephrine, epinephrine, 3,4-dihydroxyphenylacetic acid (DOPAC), isoprenaline, isoproterenol and 3,4-dihydroxybenzoic acid. In the present disclosure, dopamine hydrochloride is used as a catechol-based compound, and in this case, -NH₂ in a terminal functional group of dopamine hydrochloride may react with the biocompatible polymer (particularly, hyaluronic acid).

Also, the pyrogallol-based compound may be selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol. In the present disclosure, 5-hydroxydopamine is used as a pyrogallol-based compound, and in this case, -NH₂ in a terminal functional group of 5-hydroxydopamine may react with the biocompatible polymer (particularly, hyaluronic acid).

In particular, a pyrogallol group used as a phenol group can be naturally oxidized within minutes without treatment of an oxidizing agent when exposed to oxygen present in the body due to its rapid oxidizing properties. Therefore, when the extracellular matrix hydrogel patch is actually applied to clinical practice, it can be directly applied without treatment of an oxidizing agent.

As used herein, the term "biocompatible polymer" may react with a terminal functional group present in the phenol-based compound so as to be functionalized with the phenol group. Specifically, the biocompatible polymer may be selected from the group consisting of hyaluronic acid, heparin, cellulose, dextran, alginate, chitosan, chitin, collagen, gelatin, chondroitin sulfate, pectin, keratin and fibrin, and may be desirably hyaluronic acid and may be more desirably hyaluronic acid having a molecular weight of from 100 kDa to 10 MDa, but is not limited thereto. In that case, -COOH in a terminal functional group of hyaluronic acid may react with the phenol-based compound.

As used herein, the term "hydrogel patch" refers to a structure formed into a thin film having a predetermined thickness and including a biocompatible polymer functionalized with a phenol group, and can be cut or molded into a desired shape by a known method and thus is convenient to use. The hydrogel patch has excellent mechanical properties, tissue adhesive properties, biocompatibility and ease of use compared with a solution-based bulk hydrogel.

Specifically, the hydrogel patch may be prepared by the following processes:
(a) a process of pouring a biocompatible polymer solution functionalized with a phenol group evenly on a flat surface; and
(b) a process of freeze-drying the solution at -0.5°C to -100°C for 5 hours to 48 hours.

Specifically, the process (a) may be performed by pouring 40 µl to 200 µl of a biocompatible polymer solution functionalized with a phenol group into a cylindrical mold, and the biocompatible polymer solution functionalized with a phenol group may be used at a concentration of from 0.1 (w/v)% to 5 (w/v)% and desirably from 0.5 (w/v)% to 3 (w/v)%. The amount of the biocompatible polymer solution functionalized with a phenol group is required to form a hydrogel patch having a thickness of from 0.8 mm to 3.2 mm, and the thickness of the hydrogel patch can be easily regulated.

Further, the process (b) may be performed by freeze-drying the biocompatible polymer solution functionalized with a phenol group at -0.5°C to -100°C for 5 hours to 48 hours, or desirably by freeze-drying at -50°C to -100°C for 12 hours to 36 hours. When the biocompatible polymer solution functionalized with a phenol group is freeze-dried, the volume of the solution is decreased and a hydrogel patch in the form of a thin film having a predetermined thickness is formed.

As a result, the hydrogel patch may have i) a thickness of from 0.05 mm to 10.0 mm, desirably from 0.1 mm to 5.0 mm and more desirably from 1.6 mm to 5.0 mm, ii) a storage modulus G' of from 1×10² Pa to 1×10⁶ Pa and desirably from 1.5×10³ Pa to 1×10⁶ Pa and tanδ of from 0.2 to 0.5 in a frequency range of from 0.1 Hz to 10 Hz, iii) a friction factor of from 0.2 to 0.4 as measured at a speed of 0.01 m/s under a normal force of 5 N, and iv) an adhesive strength of from 1 N to 10 N. If the hydrogel patch is a pyrogallol group-functionalized biocompatible polymer hydrogel patch, the mechanical properties thereof can be further improved.

Also, the hydrogel patch according to the present disclosure includes the extracellular matrix loaded in the hydrogel patch. The amount of the extracellular matrix may be from 0.002 wt% to 10 wt% and desirably from 0.002 wt% to 4 wt% based on the total amount of the extracellular matrix hydrogel patch, but it is not limited thereto. In other words, 100 ng to 2 mg of the extracellular matrix can be loaded in each hydrogel patch (based on a diameter of from 0.05 mm to 10.0 mm and a thickness of from 0.05 mm to 10.0 mm and desirably based on a diameter of from 0.1 mm to 5.0 mm and a thickness of from 0.1 mm to 5.0 mm). Meanwhile, if the extracellular matrix is mixed in the process of fabricating the extracellular matrix hydrogel patch, 10 µg/ml to 500 µg/ml, specifically 20 µg/ml to 400 µg/ml and more specifically 50 µg/ml to 400 µg/ml of the extracellular matrix may be mixed when the phenol group of the hydrogel patch is catechol, and 20 µg/ml to 140 µg/ml of the extracellular matrix may be mixed when the phenol group of the hydrogel patch is gallol.

It can be effectively released in a sustained manner in vivo and thus can induce a lasting therapeutic effect. Various functional groups present in the extracellular matrix may interact with a phenol group, such as a nucleophilic reaction and a non-covalent bond. That is, the extracellular matrix may make interactions, such as nucleophilic reactions and non-covalent bonds, with the phenol group. That is, the extracellular matrix may serve as a cross-linking agent via interaction with the phenol group.

Specifically, the extracellular matrix may be derived from any one or more tissues selected from the group consisting of muscle, brain, spinal cord, tongue, airway, skin, lymph, lung, heart, liver, stomach, kidney, spleen, pancreas, intestine, adrenal gland, fat, uterus, thymus, esophagus, salivary gland, bone, bladder, blood vessel, tendon, thyroid and gum, and various functional groups present in the extracellular matrix may make interactions, such as nucleophilic reaction and non-covalent bond, with the phenol group. Thus, it can be effectively released in a sustained manner in vivo.

Specifically, a method of loading the extracellular matrix in the hydrogel patch may be performed by mixing a biocompatible polymer solution functionalized with a phenol group and an extracellular matrix to fabricate a hydrogel patch, or by applying an extracellular matrix onto a phenol group-functionalized biocompatible polymer hydrogel patch and then cross-linking the extracellular matrix to the phenol group-functionalized biocompatible polymer hydrogel patch. In this case, the extracellular matrix itself can serve as an oxidizing agent, and, thus, treatment of an oxidizing agent after the application of the extracellular matrix can be omitted. In other words, if the hydrogel patch is functionalized with a catechol group, cross-linking may occur via reaction between the catechol group and protein in the extracellular matrix only by extracellular matrix treatment without treatment of an oxidizing agent, and if the hydrogel patch is functionalized with a pyrogallol group, it is naturally oxidized in vivo without treatment of an oxidizing agent and thus is easy to use in actual clinical practice.

### Mode for Carrying out the Invention

Hereafter, one or more specific examples will be described in more detail. However, these examples are provided for illustrative purposes only and the scope of the present disclosure is not limited to these examples.

### Preparation Example 1

### (1) Fabrication of HA-CA hydrogel patch

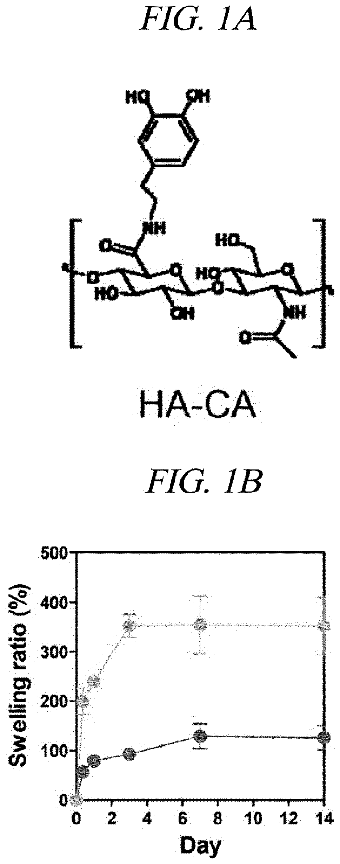

**A** catechol-functionalized hyaluronic acid with dopamine hydrochloride (hereinafter, referred to as HA-CA, **FIG. 1A**) (molecular weight=200 kDa) was dissolved in distilled water at a concentration of 1 (w/v)%, and 80 µl of the 1 (w/v)% HA-CA solution was poured into an 8 mm cylindrical mold and then freeze-dried overnight at -80°C to fabricate an HA-CA hydrogel patch with a diameter of 8 mm and a thickness of 1.6 mm. The fabricated HA-CA hydrogel patch is in a dry state and thus is easy to store. Also, it is fabricated in the form of a thin film and thus can be easily cut into a desired shape. Therefore, it is convenient to use.

Meanwhile, HA-CA was dissolved in phosphate-buffered saline (PBS), and a 4.5 mg/ml sodium periodate solution was added into the solution to fabricate an HA-CA bulk hydrogel. A final concentration of HA-CA in the fabricated HA-CA bulk hydrogel was 1 (w/v)%.

### (2) Analysis on physical properties of HA-CA hydrogel patch

The HA-CA hydrogel patch or the HA-CA bulk hydrogel was immersed in PBS at 37°C similar to in vivo conditions for 14 days, and the swelling ratio was measured after 12 hours, 1 day, 3 days, 7 days and 14 days. As a result of measurement, the swelling ratio of the HA-CA hydrogel patch (Patch) was higher than that of the HA-CA bulk hydrogel (Gel) (**FIG. 1B**).

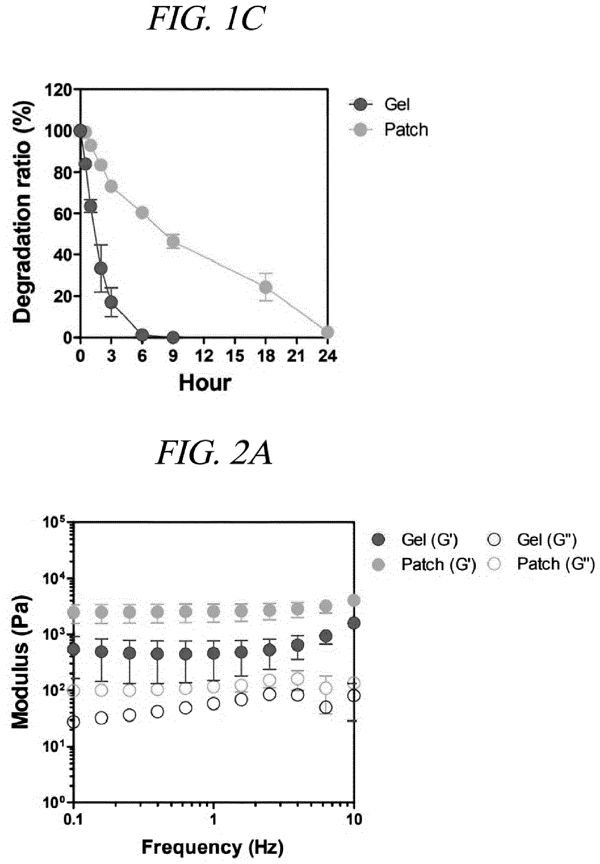

Since various degradation enzymes exist in the actual in vivo environment, the HA-CA hydrogel patch or the HA-CA bulk hydrogel was immersed in PBS at 37°C and then treated with a hyaluronidase until degradation (100 U/sample). The weight of the HA-CA hydrogel patch or the HA-CA bulk hydrogel was measured at regular time intervals to measure the degrees of degradation over time. As a result of measurement, the HA-CA bulk hydrogel (Gel) was rapidly degraded within 2 hours after the treatment with the hyaluronidase and completely degraded after 6 hours. However, the HA-CA hydrogel patch (Patch) remained even after 24 hours from hyaluronidase treatment, thus indicating a decreased enzymatic degradation rate (**FIG. 1C**).

### (3) Analysis on mechanical properties of HA-CA hydrogel patch

The elastic modulus of the HA-CA hydrogel patch or the HA-CA bulk hydrogel was measured at a frequency of from 0.1 Hz and 10 Hz by using a rheometer. As a result of analysis, both the HA-CA hydrogel patch (Patch) and the HA-CA bulk hydrogel (Gel) showed higher storage moduli G' than loss moduli G". Thus, it was found that the internal structure is formed of a stable polymer network (**FIG. 2A**).

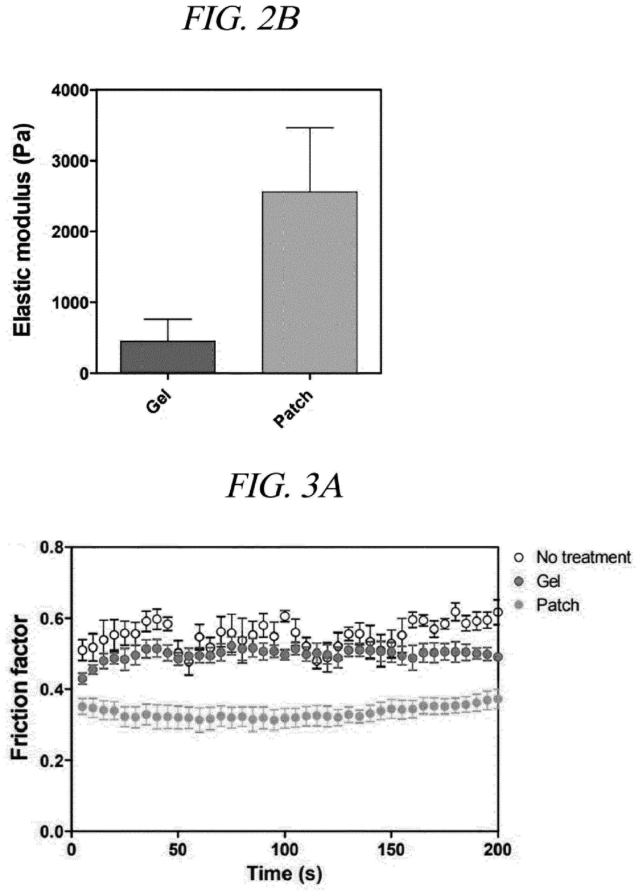

Further, the average storage modulus G' of the HA-CA bulk hydrogel (Gel) was about 450 Pa, whereas the average storage modulus G' of the HA-CA hydrogel patch (Patch) was in the range of from about 2500 Pa to about 2600 Pa. Thus, it was found that the average storage modulus G' increased by about 5 times or more (**FIG. 2B**).

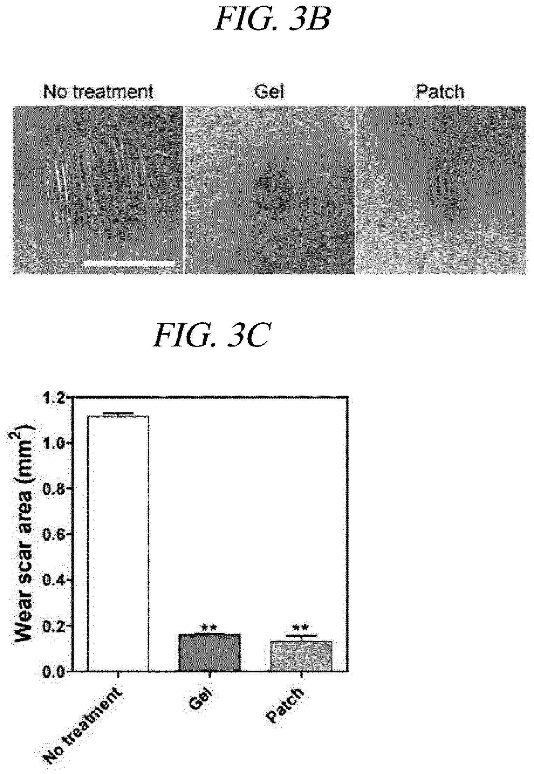

Meanwhile, a friction factor was measured by moving a friction force analyzer at a speed of 0.01 m/s in a state where a normal force of 5 N was applied between stainless steel surfaces coated with the HA-CA hydrogel patch or the HA-CA bulk hydrogel. As a result of measurement, the friction factor was the highest in the case of no coating (No treatment), followed by the HA-CA bulk hydrogel (Gel) and the HA-CA hydrogel patch (Patch) (**FIG. 3A**). Further, in the case of no coating (No treatment), a large wear scar was formed due to friction, and when the HA-CA bulk hydrogel (Gel) and the HA-CA hydrogel patch (Patch) were coated, a small wear scar was formed (**FIG. 3B**). Therefore, when the HA-CA bulk hydrogel (Gel) and the HA-CA hydrogel patch (Patch) were coated, the area of wear scar was significantly reduced compared with the case of no coating (No treatment) (**FIG. 3C**).

### Preparation Example 2

### (1) Fabrication of HA-PG hydrogel patch

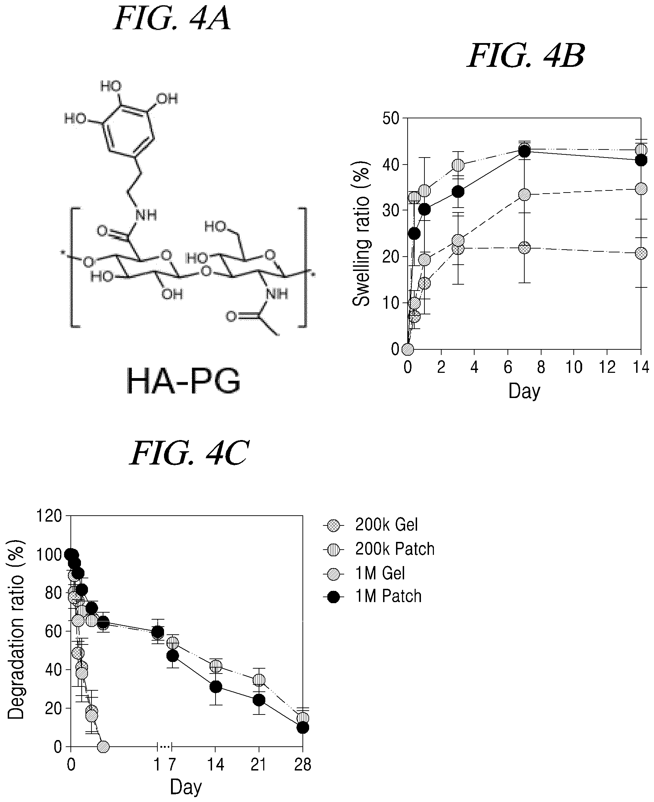

**A** pyrogallol-functionalized hyaluronic acid with 5-hydroxydopamine (hereinafter, referred to as HA-PG, **FIG. 4A**) (molecular weight=200 kDa and 1 MDa) was dissolved in distilled water at a concentration of 1 (w/v)%, and 80 µl of the 1 (w/v)% HA-PG solution was poured into an 8 mm cylindrical mold and then freeze-dried overnight at -80°C to fabricate an HA-PG hydrogel patch with a diameter of 8 mm and a thickness of 1.6 mm. The fabricated HA-PG hydrogel patch is in a dry state and thus is easy to store. Also, it is fabricated in the form of a thin film and thus can be easily cut into a desired shape. Therefore, it is convenient to use.

Meanwhile, HA-PG was dissolved in phosphate-buffered saline (PBS), and a 4.5 mg/ml sodium periodate solution was added into the solution to fabricate an HA-PG bulk hydrogel. A final concentration of HA-PG in the fabricated HA-PG bulk hydrogel was 1 (w/v)%.

### (2) Analysis on physical properties of HA-PG hydrogel patch

The HA-PG hydrogel patch or the HA-PG bulk hydrogel was immersed in PBS at 37°C similar to in vivo conditions for 14 days, and the swelling ratio was measured after 12 hours, 1 day, 3 days, 7 days and 14 days. As a result of measurement, the swelling ratio of the HA-PG hydrogel patch (Patch) was higher than that of the HA-PG bulk hydrogel (Gel) **(****FIG. 4B**).

Since various degradation enzymes exist in the actual in vivo environment, the HA-PG hydrogel patch or the HA-PG bulk hydrogel was immersed in PBS at 37°C and then treated with a hyaluronidase until degradation (200 U/sample). The weight of the HA-PG hydrogel patch or the HA-PG bulk hydrogel was measured at regular time intervals to measure the degrees of degradation over time. As a result of measurement, the HA-PG bulk hydrogels (200 kDa and 1 MDa Gels) were rapidly degraded at an early stage after the treatment with the hyaluronidase. However, the HA-PG hydrogel patches (200 kDa and 1 MDa Patches) remained even after 28 days from hyaluronidase treatment, thus indicating a considerably decreased enzymatic degradation rate **(****FIG. 4C**).

### (3) Analysis on mechanical properties of HA-PG hydrogel patch

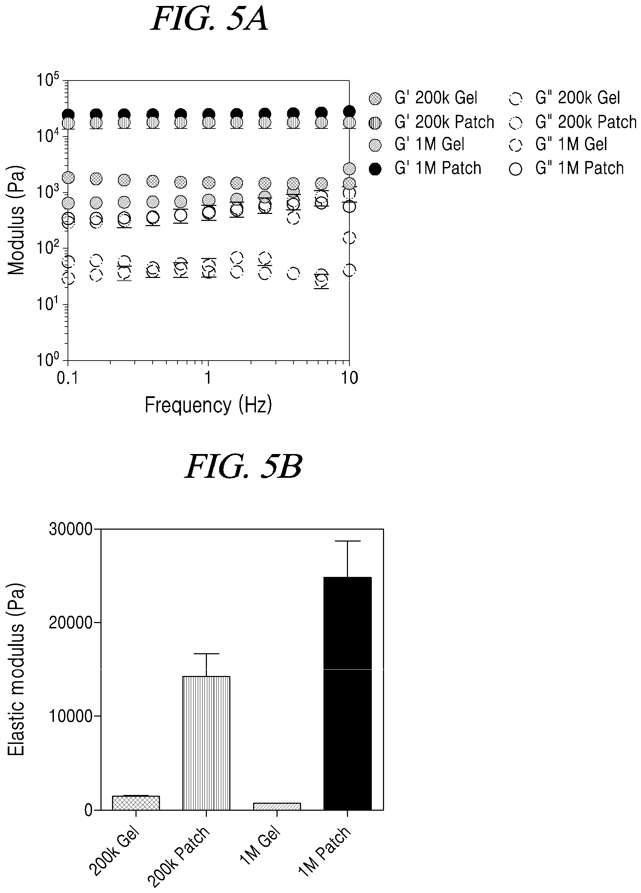

The elastic modulus of the HA-PG hydrogel patch or the HA-PG bulk hydrogel was measured at a frequency of from 0.1 Hz and 10 Hz by using a rheometer. As a result of analysis, both the HA-PG hydrogel patch (Patch) and the HA-PG bulk hydrogel (Gel) showed higher storage moduli G' than loss moduli G". Thus, it was found that the internal structure is formed of a stable polymer network **(****FIG. 5A**).

Further, the average storage moduli G' of the HA-PG bulk hydrogels (200 kDa and 1 MDa Gels) were very low, whereas the average storage moduli G' of the HA-PG hydrogel patches (200 kDa and 1 MDa Patches) were in the range of from about 14 kPa to about 24 kPa. Thus, it was found that the average storage moduli G' increased considerably (**FIG. 5B**).

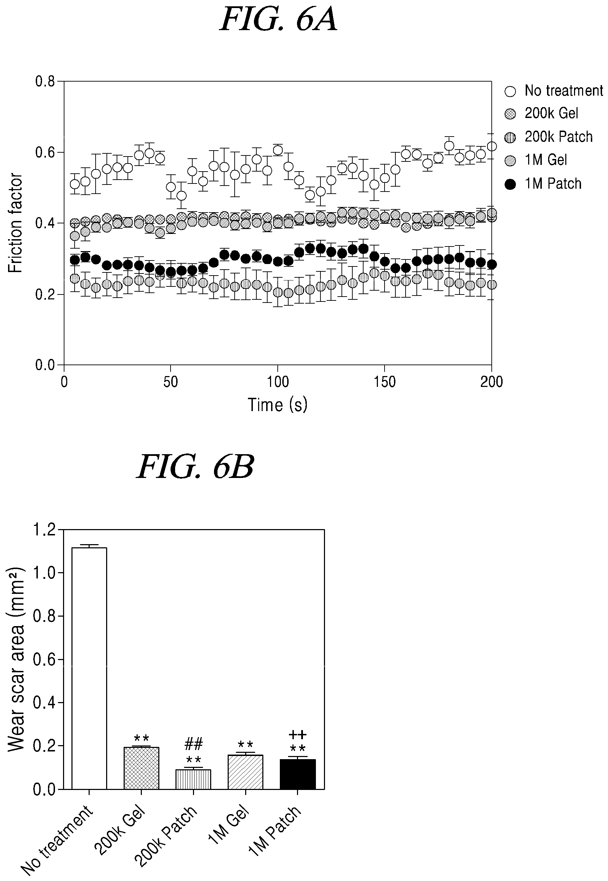

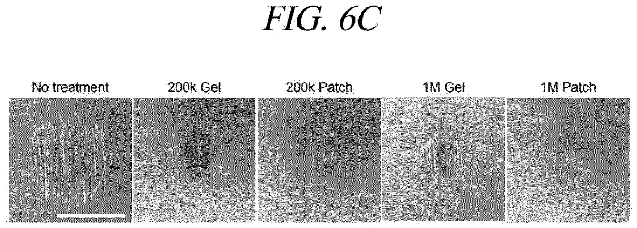

Meanwhile, a friction factor was measured by moving a friction force analyzer at a speed of 0.01 m/s in a state where a normal force of 5 N was applied between stainless steel surfaces coated with the HA-PG hydrogel patch or the HA-PG bulk hydrogel. As a result of measurement, the friction factor was the highest in the case of no coating (No treatment), followed by the HA-PG bulk hydrogels (200 kDa and 1 MDa Gels) and the HA-PG hydrogel patches (200 kDa and 1 MDa Patches) (**FIG. 6A**). Further, in the case of no coating (No treatment), a large wear scar was formed due to friction, and when the HA-PG bulk hydrogels (200 kDa and 1 MDa Gels) and the HA-PG hydrogel patches (200 kDa and 1 MDa Patches) were coated, a small wear scar was formed (**FIG. 6B**). Therefore, when the HA-PG bulk hydrogels (200 kDa and 1 MDa Gels) and the HA-PG hydrogel patches (200 kDa and 1 MDa Patches) were coated, the area of wear scar was significantly reduced compared with the case of no coating (No treatment) (**FIG. 6C**).

### Example 1: Fabrication and analysis of phenol group-functionalized hydrogel patch for tissue-derived extracellular matrix (ECM) delivery (1)

### 1-1. Fabrication of phenol group (catechol group)-functionalized hydrogel patch (MEM/HA-CA) for muscle extracellular matrix (MEM) delivery

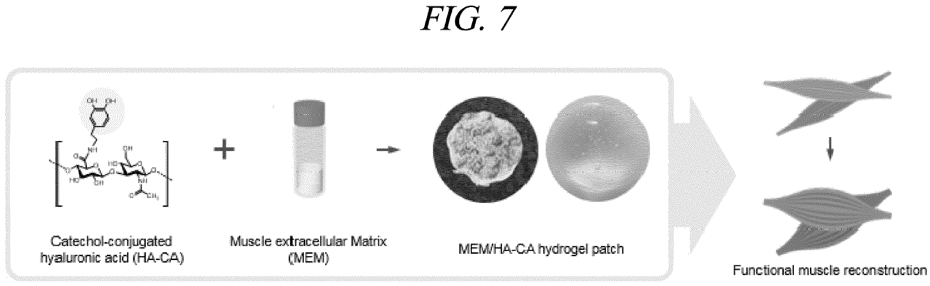

A catechol-conjugated hyaluronic acid (HA-CA) derivative loaded with a decellularized muscle extracellular matrix (MEM) was freeze-dried to fabricate a MEM/HA-CA patch formulation. The developed MEM/HA-CA patch is a new system that can be cross-linked without the addition of an oxidizing agent and has excellent biosafety. When it is fabricated in the form of a freeze-dried patch, it is easy to use and can be stored for a long time. Also, it can be used as a functional biomaterial that can induce muscle disease treatment and tissue regeneration by using various physiologically active materials and tissue-specific proteins present in the MEM (**FIG. 7**).

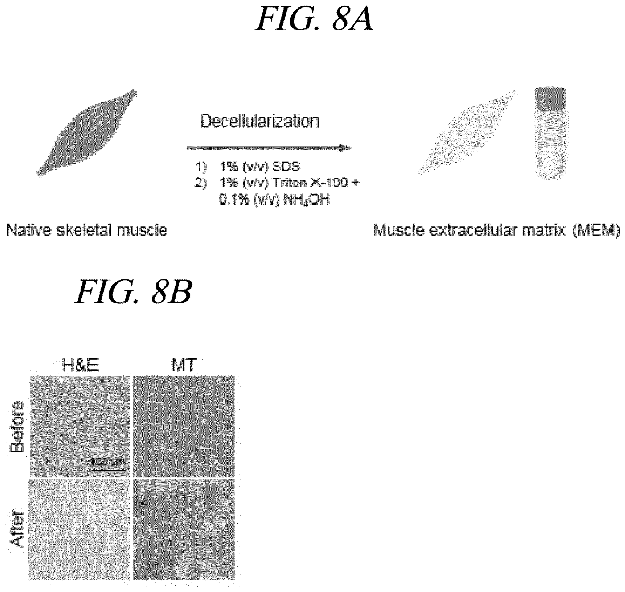

The decellularized muscle extracellular matrix used herein was fabricated through decellularization including treating porcine leg muscle with 1% sodium dodecyl sulfate (SDS) for 2 days and then treating the porcine leg muscle with 1% Triton X-100 + 0.1% NH₄OH (ammonium hydroxide) solution for 2 hours (**FIG. 8A**).

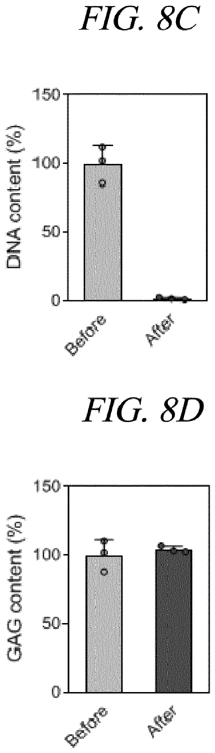

The histological morphology (**FIG. 8B**) of the muscle tissue before decellularization (Before) and after decellularization (After), the DNA content (**FIG. 8C**), and the GAG (glycosaminoglycan) content (**FIG. 8D**) were quantitatively compared. By removing 98.4% of the genetic material (DNA) through decellularization, immune responses that can occur when applied to the body were minimized, whereas GAG, a representative active extracellular matrix component, was maintained at the original tissue level. Thus, it was confirmed that a decellularization protocol for the muscle tissue was successfully established.

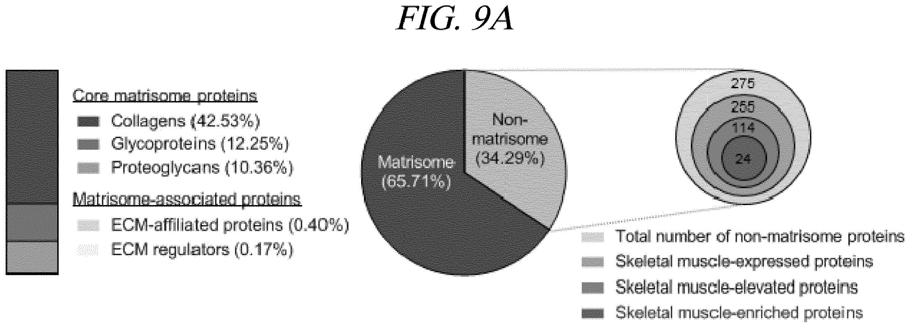

Various types of muscle-specific proteins present in the decellularized muscle extracellular matrix (MEM) were identified through mass spectrometry-based proteomics and protein content analysis using the intensity-based absolute quantification (iBAQ) algorithm **(****FIGS. 9A** to **9D****).**

As shown in **FIG. 9A****,** the MEM consists of 65.71% core matrisome proteins composed of collagen, glycoprotein and proteoglycan, and matrisome-associated proteins, and 34.29% non-matrisome proteins, making a total of 275 proteins. It was confirmed that 255 of them are muscle-specific proteins.

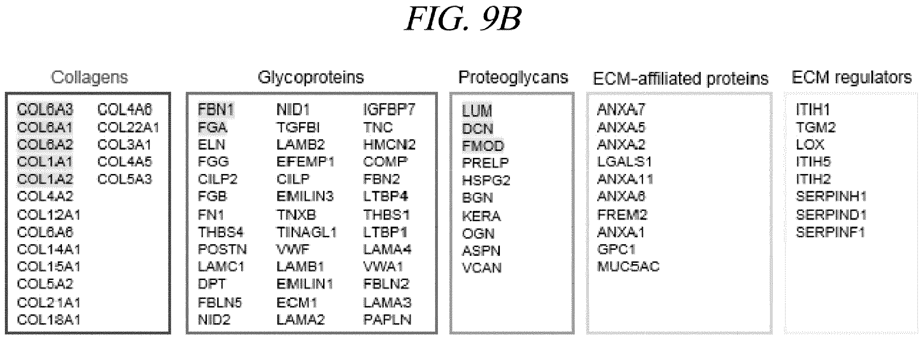

The matrisome proteins of the MEM are composed of 18 collagens, 39 glycoproteins, 10 proteoglycans, 10 ECM-affiliated proteins and 8 ECM regulators classified by type. The top 10 matrisome proteins with high expression levels (highlighted in yellow) are collagen 6 (COL6A3, COL6A1, COL6A2), collagen 1 (COL1A1, COL1A2), fibrillin (FBN1), fibrinogen (FGA), lumican (LUM), decorin (DCN) and fibromodulin (FMOD), which have been reported to be related to muscle regeneration. Thus, the MEM is considered to be suitable for inducing the regeneration and maturation of damaged muscle tissue (**FIG. 9B**).

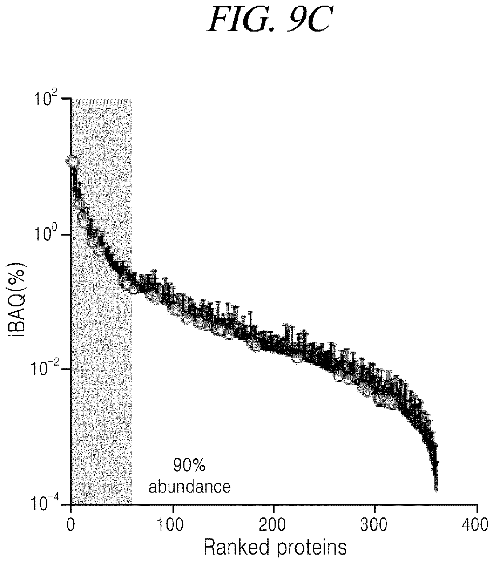

All the proteins constituting the MEM were mapped according to the iBAQ values to check the distribution. The collagens, which occupy the largest proportion of the MEM, are indicated in red, and skeletal muscle-enriched proteins with expression levels at least four times higher in the muscle tissue than in other tissues are indicated in blue. In the MEM, 61 proteins account for 90% of the total weight (gray area), and of these, a total of 27 matrisome proteins were identified. The 27 matrisome proteins are composed of 9 collagens, 11 glycoproteins, 6 proteoglycans and 1 ECM-affiliated protein, and the remaining 34 non-matrisome proteins are composed of 25 skeletal muscle-elevated proteins and 6 skeletal muscle-enriched proteins (**FIG. 9C**).

Both the skeletal muscle-elevated protein and the skeletal muscle-enriched protein refer to proteins with average expression levels four or more times higher in the muscle tissue than in other tissues according to information provided by "https://www.proteinatlas.org/" that analyzes protein expression patterns in human tissues. However, the skeletal muscle-enriched protein is a protein observed to be expressed at an expression level absolutely four or more times higher in the muscle tissue, and, thus, the classification of the skeletal muscle-enriched protein is more meaningful classification than that of the skeletal muscle-elevated protein.

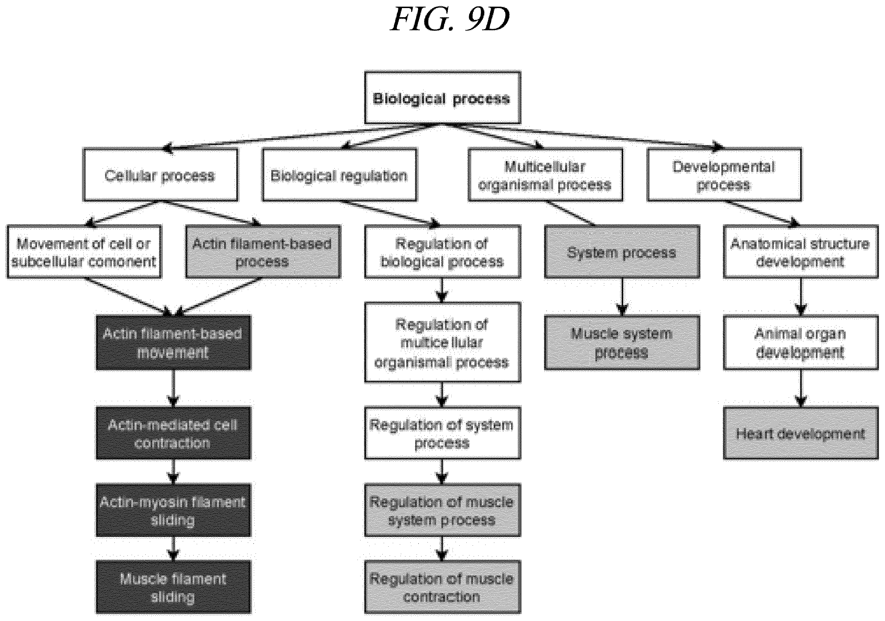

It was confirmed that when Gene Ontology (GO) analysis of 275 non-matrisome proteins except for the matrisome proteins was performed to predict the function of the MEM in terms of biological process, GO terms such as actin filament-based process (GO:0030029), regulation of muscle system process (GO:0090257), muscle system process (GO:0003012) and their related sub-items were predicted at a significant level (enrichment p value < 10 ⁻³) (**FIG. 9D**).

The GO analysis was performed using the web-based GOrilla application (http://cbl-gorilla.cs.technion.ac.il).

It was found through a series of proteomic analyses that a large amount of tissue-specific proteins present in actual muscle tissue is present in the MEM. Thus, the MEM is considered to be utilized as an active material for muscle disease treatment and tissue regeneration.

### 1-2. Analysis on chemical cross-linking mechanism of MEM/HA-CA hydrogel patch

Various proteins present in the MEM are expected to react with HA-CA to induce cross-linking.

To verify this, comparative analysis was performed by UV-vis spectroscopy while a solution including HA-CA (0.5%) with the MEM (7 mg/ml) (MEM/HA-CA) and a solution including HA-CA (0.5%) without the MEM (7 mg/ml) (HA-CA) were incubated at 37°C.

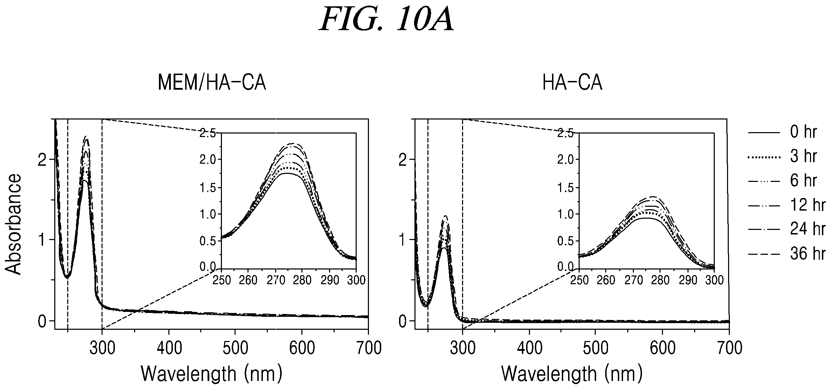

As a result, the 280 nm peak for the HA-CA group increased, which indicates that covalent bonds between catechols (e.g., dicatechol) were formed by natural oxidation. Further, the 280 nm peak for the MEM/HA-CA group sharply increased compared with that for HA-CA group. Therefore, it can be seen that the oxidation of catechol was promoted by the MEM, and, thus, the formation of catechol-catechol covalent bonds was promoted. Here, a new peak was not significantly observed in a range of from 450 nm to 550 nm, and, thus, it seems that catechol and the MEM (e.g., catechol and amine) did not form a large number of covalent bonds and it is presumed that the HA-CA derivative and the MEM formed a large number of non-covalent bonds (e.g., hydrogen bonds) (**FIG. 10A**).

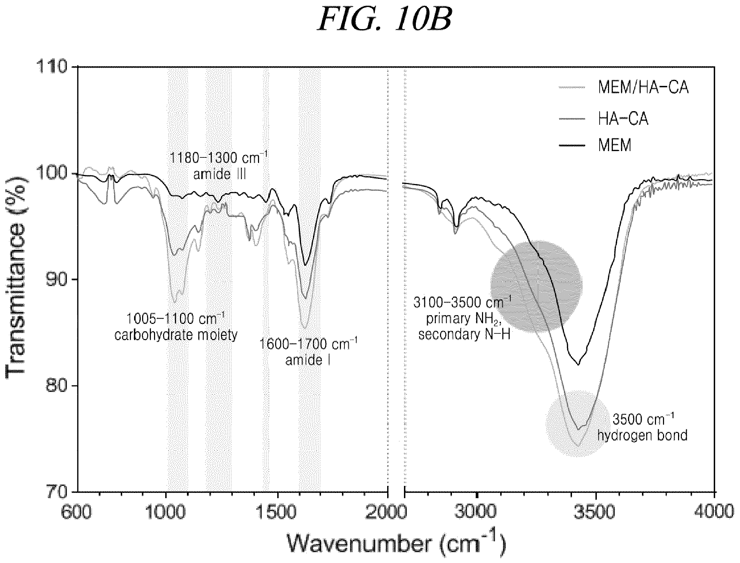

The result of FT-IR analysis shows peak changes of MEM protein (amide bond; 1600 cm⁻¹ to 1700 cm⁻¹, 1180 cm⁻¹ to 1300 cm⁻¹, -CH₃ & -CH₂ group; 1453 cm⁻¹, carbohydrate moiety; 1005 cm⁻¹ to 1100 cm⁻¹, 1164 cm⁻¹), which confirms that an interaction occurred between the HA-CA derivative and the MEM (**FIG. 10B**). Here, the formation of primary NH₂ and secondary N-H stretching (blue circle, 3100 cm⁻¹ to 3500 cm cm⁻¹) and a large number of hydrogen bonds (green circle, 3500 cm⁻¹) was observed.

Therefore, cross-linking of the MEM/HA-CA mainly occurred through the formation of covalent bonds between catechol groups (e.g., formation of dicatechol) and the formation of non-covalent bonds between the HA-CA and the MEM(e.g., formation of hydrogen bonds). Here, amide bonds supplied from a large amount of protein introduced from the MEM are considered to enhance the stability of the three-dimensional hydrogel.

### 1-3. Analysis on mechanical properties and adhesive properties of MEM/HA-CA hydrogel patch

HA-CA derivatives originally need to be treated with an oxidizing agent to induce stable cross-linking which occurs only when catechol groups are oxidized. However, if the MEM is applied, stable cross-linking can be induced by covalent bonds between catechols and a large number of hydrogen bonds between the HA-CA derivative and the MEM even without treatment of an oxidizing agent. A case where an oxidizing agent (NaIO₄ group; 4.5 mg/ml NaIO₄) was added to induce cross-linking of the HA-CA as mainly used in previous studies was set as a control group for comparison. In all groups except the MEM only group, 0.5% HA-CA (DS: 15%) was used.

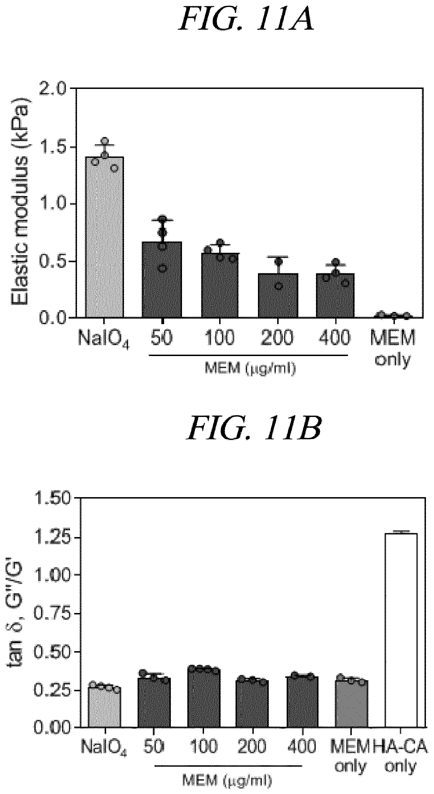

It was confirmed through rheometric analysis (frequency sweep mode) that the physical properties were decreased as the concentration of the added MEM increased. Thus, it was confirmed that the physical properties of the MEM/HA-CA hydrogel patch can be controlled depending on changes in concentration of the MEM (**FIG. 11A**).

All the groups added with the MEM have a tanδ value (elasticity) of 0.39 or less, which confirms that a stable hydrogel was formed. Meanwhile, the HA-CA group treated only with tertiary distilled water without the MEM has an average elasticity value of 1.27, which confirms once again that cross-linking of the HA-CA derivative was induced by the MEM component (**FIG. 11B**).

When the elasticity value is 1 or more, a G" (loss modulus) value is relatively greater than a G' (storage modulus) value, so it cannot be a hydrogel.

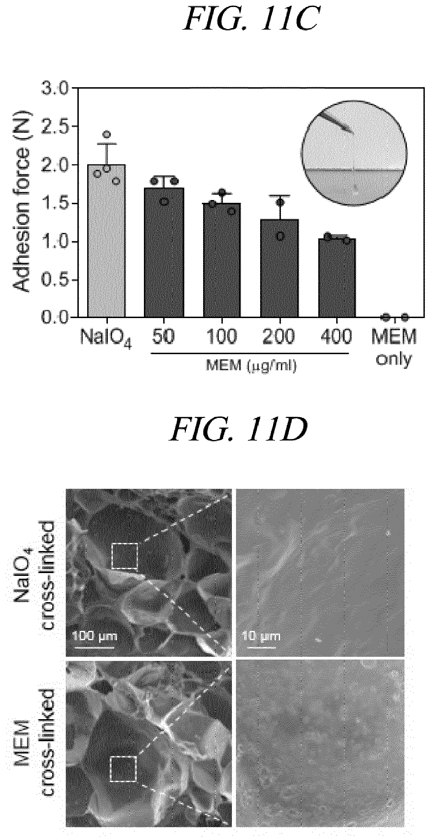

It was confirmed through rheometric analysis (tack test mode) that the adhesive properties of the MEM/HA-CA hydrogel patch showed a tendency to decrease as the concentration of MEM increased, but was 19.5 to 33 times higher than that of a conventional MEM hydrogel (MEM only group; 5 mg/ml MEM hydrogel) (**FIG. 11C**).

As a result of analyzing the internal structure of the hydrogel patch by using a scanning electron microscopy (SEM) analysis, no change in pore size depending on the cross-linking method (cross-linking with NaIO₄ according to the prior art vs cross-linking with MEM according to the present disclosure) was observed, which confirms similarity in the internal porous structure (**FIG. 11D**).

To check whether there is a difference in the mechanical properties of a hydrogel formed at the time of addition of the MEM when the HA-CA was cross-linked using the MEM, a group added with the MEM (pre-mixed) before fabrication of the HA-CA patch and a group added with the MEM (added later) after fabrication of the HA-CA patch were compared in terms of modulus. As for the pre-mixed group, the MEM was previously mixed with the HA-CA solution and then freeze-dried to fabricate a patch. As for the added later group, the HA-CA solution was freeze-dried to fabricate a patch and then the MEM was added later when a hydrogel was formed.

The physical properties of hydrogels were compared under various cross-linking conditions and MEM concentration conditions to make final MEM concentrations of 50 µg/ml, 100 µg/ml, 200 µg/ml and 400 µg/ml (groups 50, 100, 200 and 400, respectively) in a 0.5% HA-CA (DS: 15%) solution.

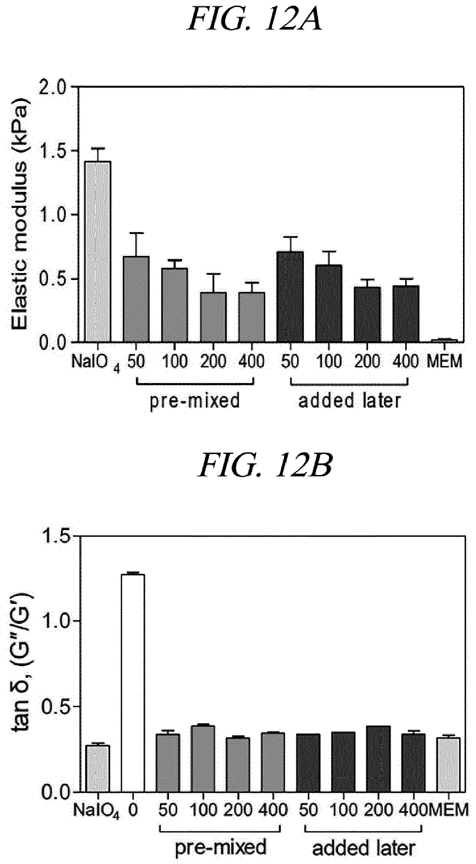

It was confirmed that a hydrogel having an elastic modulus of about 400 Pa to about 700 Pa was formed regardless of the time of addition of the MEM. Although the elastic modulus was reduced to 50% or less compared with when the HA-CA was cross-linked by using an oxidizing agent NaIO₄, the physical properties were enhanced up to 30 times compared with those of the conventional MEM hydrogel (MEM hydrogel having a concentration of 5 mg/ml, which is the most commonly used concentration) (**FIG. 12A**).

The HA-CA without the MEM (group 0) had a tan delta value of 1 or more, which confirms that only the HA-CA derivative itself cannot form a hydrogel without the MEM (**FIG. 12B**).

### 1-4. Analysis on physical properties (swelling and degradation patterns) of MEM/HA-CA hydrogel patch

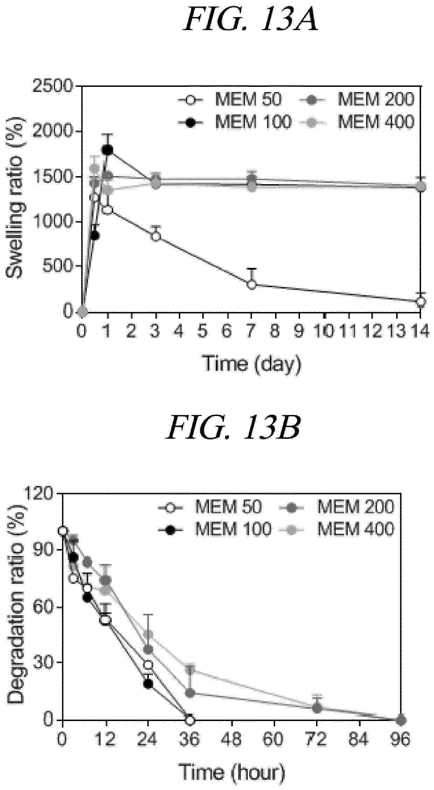

The swelling patterns of the MEM/HA-CA hydrogel patches were compared under various MEM concentration conditions (37°C, PBS incubation, 0.5% HA-CA). It was confirmed that in the MEM 50 µg/ml group with the lowest MEM concentration, the hydrogel structure swollen over time was not well maintained, but under MEM concentration conditions of 100 µg/ml or more (100 µg/ml, 200 µg/ml and 400 µg/ml groups), the swollen structure of the hydrogel was well maintained **(****FIG. 13A****).**

The degradation pattern of the MEM/HA-CA hydrogel patch was analyzed (37°C, 2.5 U/ml HAdase incubation). It was confirmed that the degradation rate decreased as the concentration of the added MEM increased. Thus, it can be seen that the in vivo degradation rate of the hydrogel can be controlled depending on the MEM concentration (**FIG. 13B**).

### 1-5. Check of possibility of sustained MEM delivery by MEM/HA-CA hydrogel patch

Reversible reactions are carried out by hydrogen bonds, i.e., non-covalent bonds, formed between the MEM and the HA-CA derivative, and, thus, the MEM/HA-CA hydrogel patch is expected to show a sustained-release behavior over time without damage to the activity of the MEM protein, which was checked.

Specifically, the MEM was loaded in the HA-CA hydrogel patch (400 µg/ml MEM + 0.5% HA-CA) and cross-linking was induced with the MEM or NaIO₄ (4.5 mg/ml NaIO₄). Then, the release pattern was checked (37°C, PBS incubation).

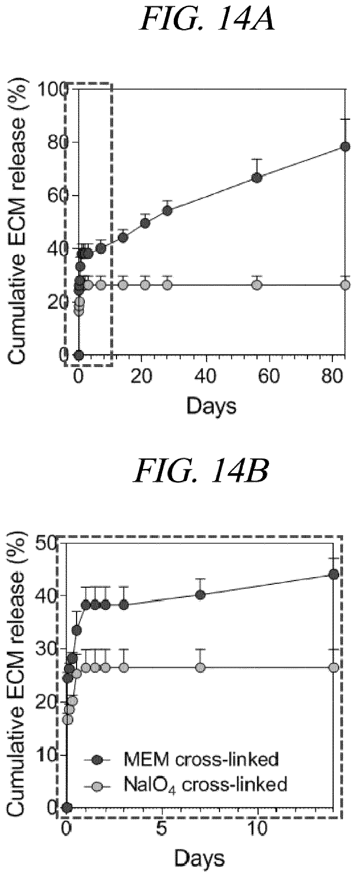

**As** a result, it was confirmed that the hydrogel patch cross-linked with the MEM (MEM cross-linked) was gradually released over 3 months (**FIG. 14A**). Also, it was confirmed that the hydrogel patch (NaIO₄ cross-linked) cross-linked by NaIO₄ treatment released little MEM due to strong covalent bonds between the oxidized catechol and the MEM protein after a small amount of the MEM was initially released (**FIG. 14B**).

Therefore, it was confirmed that the MEM/HA-CA hydrogel patch can be used as a sustained-release formulation for delivering muscle ECM.

### 1-6. Check of biocompatibility of MEM/HA-CA hydrogel patch

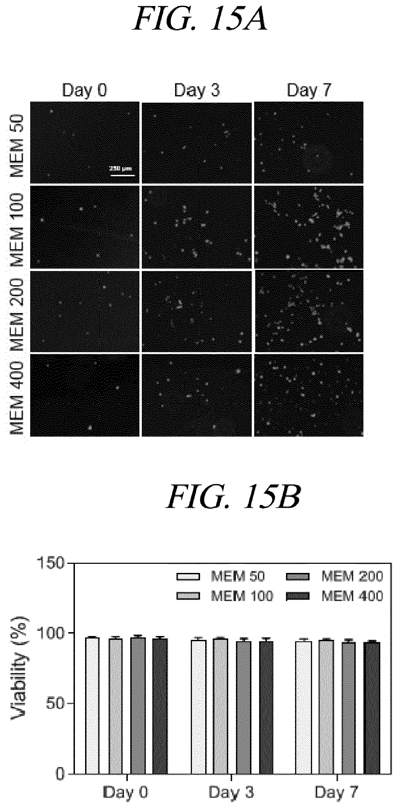

Satellite cells called muscle stem cells were isolated from mouse thigh muscle tissue and seeded at a concentration of 5×10⁴ cells/gel in HA-CA hydrogel patches (0.5% HA-CA) cross-linked under various MEM concentration conditions. As a result of performing live/dead assay while the HA-CA hydrogel patches were incubated for 7 days, the MEM/HA-CA hydrogel patches showed a cell viability of 92.9% or more regardless of the MEM concentration, which confirms that the MEM/HA-CA hydrogel patches have little cytotoxicity (**FIGS. 15A** and **15B**).

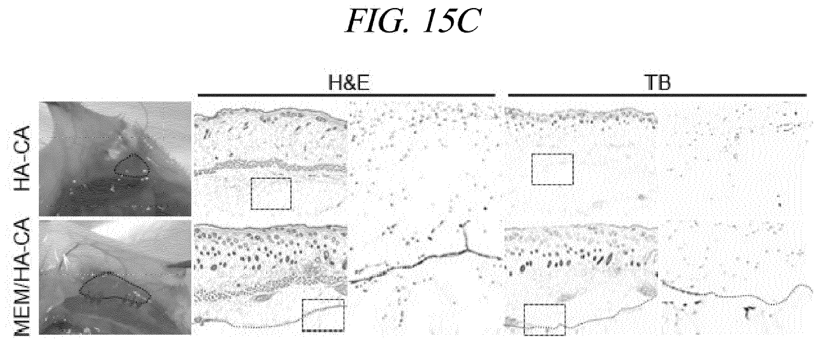

Also, when the hydrogel patches were checked 1 day after the engraftment of the MEM/HA-CA patch under the mouse skin, it was confirmed that the MEM/HA-CA group was well attached to the engraftment site (**FIG. 15C****,** red arrows). Further, the result of histological analysis by hematoxylin & eosin (H&E) staining and toluidine blue (TB) staining confirmed that the engrafted MEM/HA-CA was well maintained without inducing a special immune response or toxicity in the body (**FIG. 15C****,** area under the black dotted line in the H&E and TB-stained images of the MEM/HA-CA group). However, in the group (HA-CA) engrafted with the HA-CA only without a cross-linking agent, any hydrogel structure was not formed and most of the engrafted materials disappeared, and it was difficult to find the hydrogel structure even by histological analysis (**FIG. 15C**). Accordingly, it can be seen that only the HA-CA derivative itself cannot form a hydrogel even when exposed to in vivo oxidation conditions.

Both the MEM/HA-CA and the HA-CA were engrafted in the form of a freeze-dried patch (cross-linking agent was not applied).

**Therefore,** it was confirmed that the MEM/HA-CA hydrogel patch developed in the present disclosure has very excellent tissue adhesive properties and biocompatibility and is a very easy and safe material for actual in vivo application.

### 1-7. Check of effect of enhancing muscle stem cell activation by MEM/HA-CA hydrogel patch

Satellite cells, called muscle stem cells, were isolated from mouse thigh muscle tissue and incubated in an MEM/HA-CA hydrogel patch (50 µg/ml, 100 µg/ml, 200 µg/ml or 400 µg/ml MEM in 0.5% HA-CA). Then, the expression levels of a satellite cell-specific marker Pax7 (Paired Box 7) and activated satellite cell-specific markers MyoD (myoblast determination protein 1) and desmin were checked by quantitative PCR analysis and cell immunostaining. Since the satellite cells can form myofibers through differentiation, the expression levels of MyoG (myogenic factor 4, Myogenin), which is a marker expressed during differentiation, formation and reconstruction of volumetric muscle, and MF20 (MYH1E), which is a myosin heavy chain marker, were also analyzed.

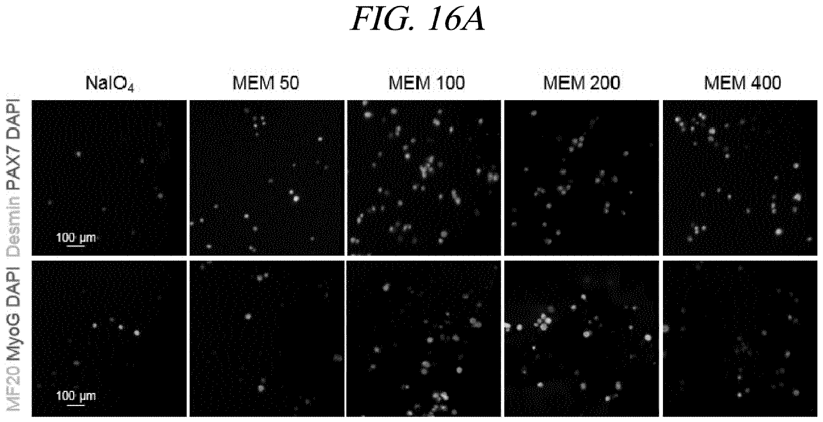

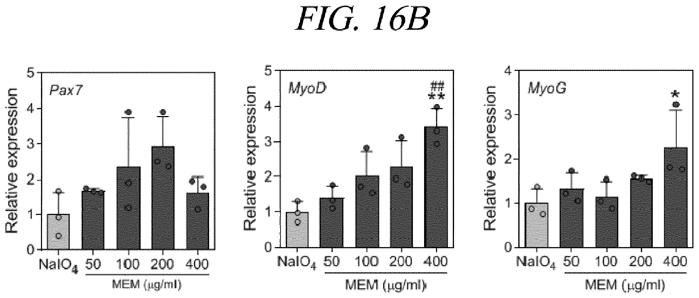

**As** a result of cell immunostaining on day 1 of incubation, it was confirmed that the expression level of the markers Pax7 and desmin increased as the concentration of the MEM increased, which confirms that the satellite cells were activated (**FIG. 16A**). Since it was at an early stage of incubation, expression of the differentiation markers MF20 and MyoG was hardly observed in all of the groups. The gene expression pattern was checked through quantitative PCR on day 1 of incubation. As the concentration of the MEM increased, the expression level of Pax7 and MyoD tended to increase. Particularly, the MEM 400 group showed a low expression level of Pax7 and the highest expression level of MyoG (**FIG. 16B**). It is considered that the stemness of the satellite cells is decreased and differentiation thereof is induced when the MEM concentration increases to a certain level or more.

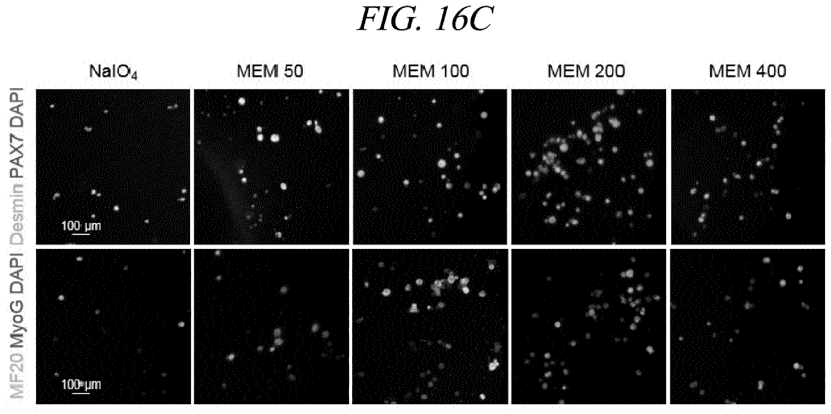

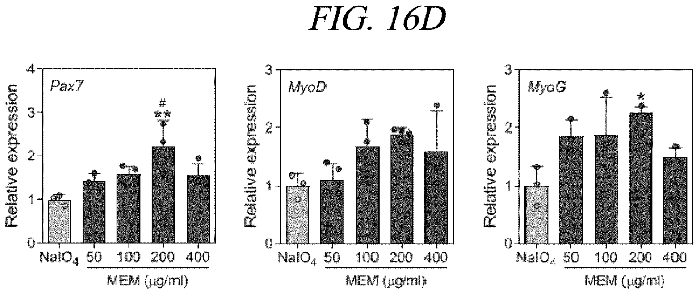

On day 3 of incubation, in the MEM 200 group, cell proliferation occurred most and the markers Pax7 and desmin were expressed in most of the cells. Accordingly, it seems that the activation of the satellite cells progressed significantly. It was confirmed that the differentiation markers MF20 and MyoG were partially increased in the MEM 100 and MEM 200 groups (**FIG. 16C**). On day 3 of incubation, the gene expression of each of Pax7, MyoD and MyoG tended to increase as the concentration of the MEM increased, and the expression decreased in the MEM 400 group to which the highest concentration of the MEM was added (**FIG. 16D**). On days 1 and 3 of incubation, in all the groups added with the MEM, cell proliferation and the levels of the related gene expression were enhanced compared to the control group in which HA-CA crosslinking was induced by NaIO4 (NaIO4) (**FIGS. 16A** to **16D**)**.** The addition of the MEM is considered to enhance the proliferation and activity of the satellite cells.

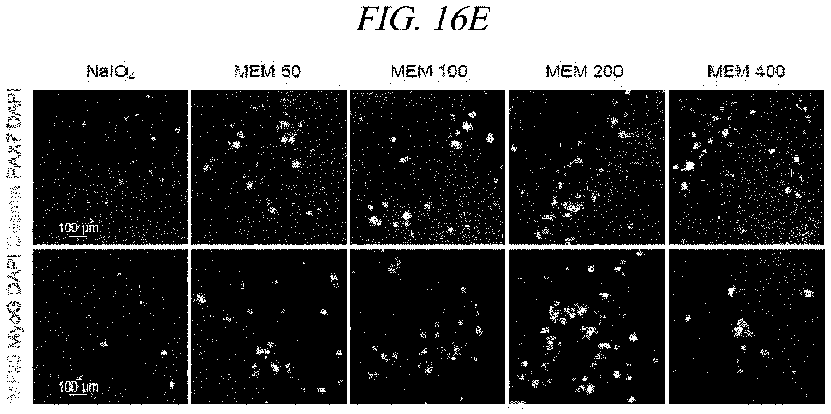

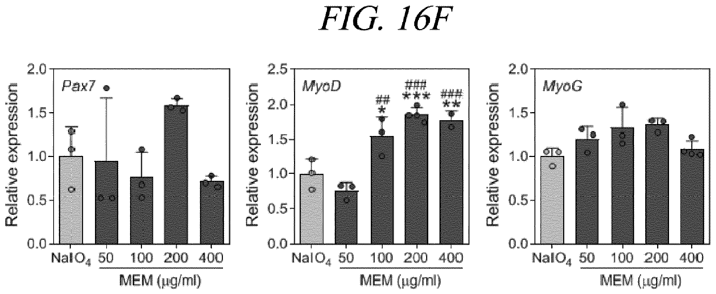

On day 7 of incubation, the MEM 50, MEM 100 and MEM 200 groups showed similar expression levels of the markers Pax7 and desmin (**FIG. 16E**). The MEM 200 group showed the highest expression levels of the differentiation markers MF20 and MyoG. On day 7 of incubation, the expression level of Pax7 was still high in the MEM 200 group, but was slightly lower in the other groups than in the NaIO₄ group (**FIG. 16F**). The expression level of the marker MyoD was more than 1.52 times higher in all the groups added with the MEM of 100 µg/ml or more than in the NaIO₄ group, and the expression level of MyoG was found to increase up to the MEM 200 group as the concentration of the MEM increased. Therefore, the satellite cell differentiation is considered to take place in the groups added with the MEM of 100 µg/ml or more. Among them, the HA-CA hydrogel patch fabricated using 200 µg/ml MEM (MEM 200) can maintain the activation of the satellite cells (Pax7+, MyoD+) for the longest period of time and can also be expected to have an appropriate differentiation effect, and, thus, it was identified as the most suitable condition for muscle regeneration.

### 1-8. Check of effect of regenerating muscle tissue by MEM/HA-CA hydrogel patch in volumetric muscle loss model

The HA-CA hydrogel patch (MEM/HA-CA) loaded with the MEM (200 µg/ml) was applied to a volumetric muscle loss model (VML model) to check its potential as a therapeutic agent for muscle tissue regeneration.

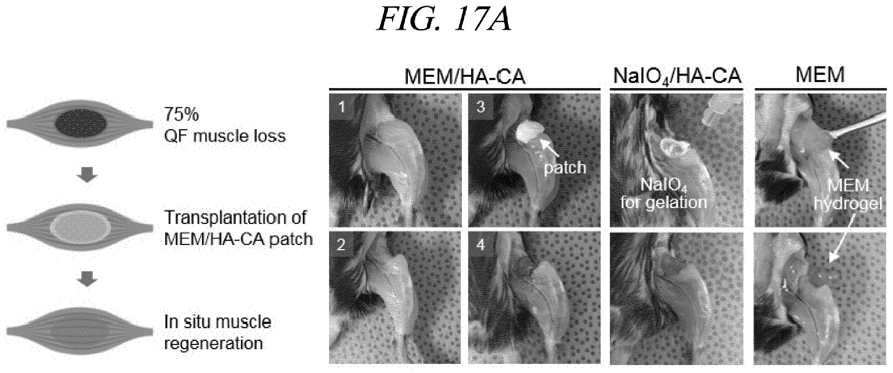

The MEM/HA-CA hydrogel patch was easily attached and fixed to the damaged tissue without a separate medical adhesive due to its excellent tissue adhesive properties. However, according to the conventional method (NaIO_{4/}HA-CA) that requires cross-linking using an oxidizing agent, NaIO₄ needs to be separately injected after the patch is applied, followed by waiting until the HA-CA is oxidized and then the surplus oxidizing agent needs to be washed. Meanwhile, the MEM hydrogel (5 mg/ml MEM) had no adhesive properties and thus was not properly fixed to the muscle damage site (**FIG. 17A**).

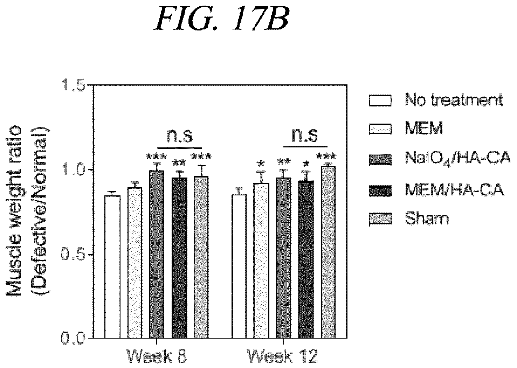

After muscle damage was induced by removing 75% of the quadriceps femoris muscle, hydrogels under various conditions were applied. Then, the damaged tissue was extracted at 8 and 12 weeks and compared in weight with the normal tissue (the same subject's opposite leg to which damage was not induced). As a result, the MEM/HA-CA and NaIO₄/HA-CA groups did not have a significant difference from the normal group (sham), which confirms that they achieved tissue mass recovery to normal level (**FIG. 17B**).

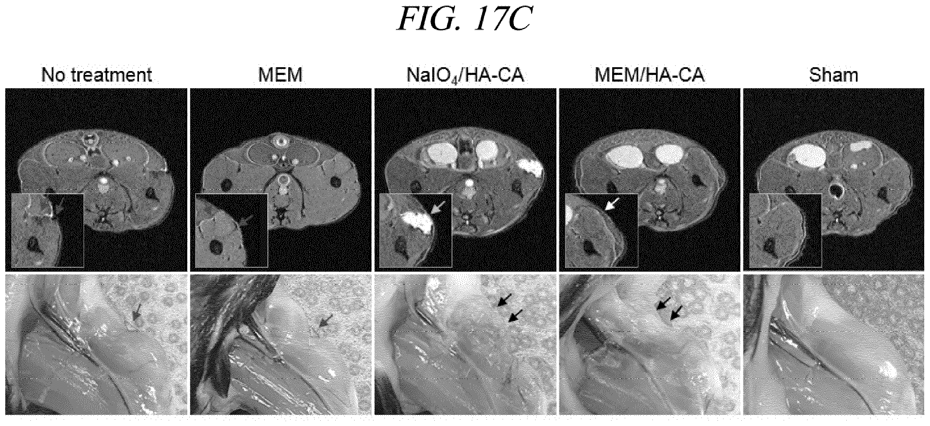

At 12 weeks, MRI (T2-image) was performed to check how much muscle tissue had been regenerated. As a result, sufficient muscle tissue regeneration was not observed in the group without any treatment (no treatment) and the MEM group after damage induction (red arrows). However, in the NaIO₄/HA-CA group, muscle tissue regeneration of sufficient size was observed, but highlighted in white due to its high water content. Therefore, it was confirmed that the recovered muscle tissue was not integrated rapidly with the surrounding tissue due to its relatively hard physical properties and still had a high hydrogel content (**FIG. 17C****,** yellow arrow). Meanwhile, in the MEM/HA-CA group, the recovered muscle tissue was well integrated with the surrounding tissue and tissue regeneration of sufficient size was observed (white arrow), which was observable with the naked eye (black arrows: regenerated muscle tissue by the engrafted hydrogel patch) (**FIG. 17C**).

In conclusion, it was confirmed that engraftment treatment using an MEM/HA-CA hydrogel patch promotes the proliferation and differentiation of stem cells present in the muscle through efficient delivery of a muscle extracellular matrix (MEM) and development of a muscle microenvironment and thus can induce tissue regeneration to normal muscle level in a volumetric muscle loss animal model.

### 1-9. Check of effect of improving muscle function by MEM/HA-CA hydrogel patch in volumetric muscle loss model

After the engraftment of the MEM/HA-CA hydrogel patch, the motor function of a volumetric muscle loss animal model was evaluated with the Rota-rod.

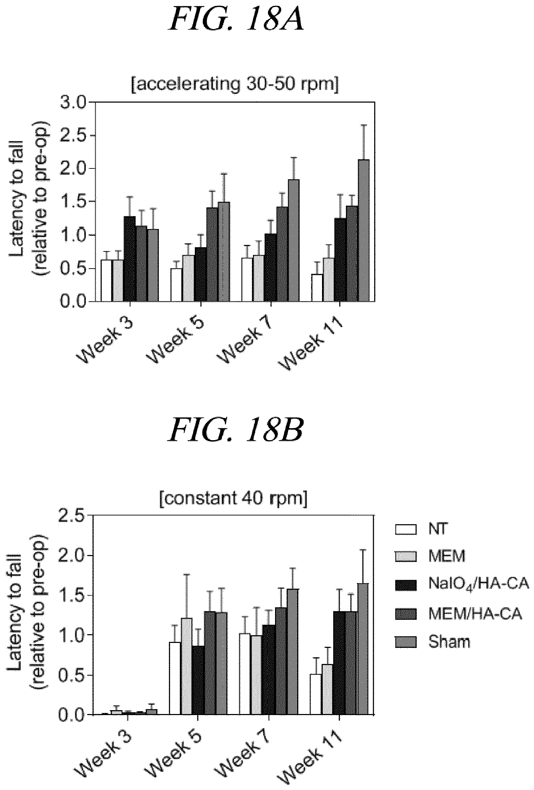

**As** a result, the MEM, NaIO₄/HA-CA and MEM/HA groups exhibited improved motor function when the Rota-rod was accelerated at 30 rpm to 50 rpm (**FIG. 18A**) or maintained at 40 rpm **(****FIG. 18B****).** In particular, the MEM/HA-CA group function. Therefore, it was confirmed that treatment using an MEM/HA-CA hydrogel patch can induce the regeneration of functionally improved muscle in a volumetric muscle loss animal model.

### 1-10. Check of effect of muscle tissue regeneration by MEM/HA-CA hydrogel patch in volumetric muscle loss model

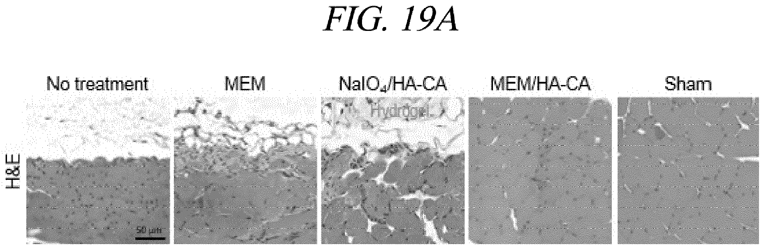

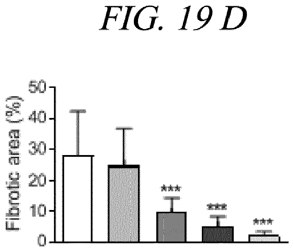

At 12 weeks after the engraftment of the MEM/HA-CA hydrogel patch, any regenerated muscle tissue was not observed in the group without any treatment (no treatment) and the group engrafted with the MEM hydrogel only (MEM, 5 mg/ml MEM hydrogel). In the group engrafted with the HA-CA patch cross-linked with NaIO₄ (NaIO₄/HA-CA, 0.5% HA-CA) and the group engrafted with the HA-CA patch cross-linked with the MEM (MEM/hydrogel patch loaded with 200 µg/ml MEM in 0.5% HA-CA), tissue was newly formed, but in the NaIO₄/HA-CA group, the hydrogel remained as it was, which confirms that substantial regeneration of muscle tissue did not occur (**FIG. 19A**).

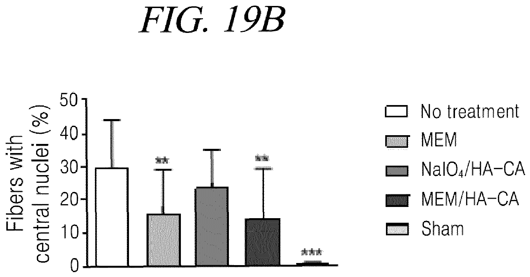

As a result of analyzing the newly formed tissue, small myotubes and dystrophic myofibers with nuclei in the center were mixed in the No treatment group and the NaIO₄/HA-CA group, which confirms that the damaged muscle was partially regenerated. Meanwhile, the MEM/HA-CA group was found to have most of the nuclei outside myofibers as in the normal tissue (sham group), which confirms that tissue regeneration was almost completed (**FIG. 19B**).

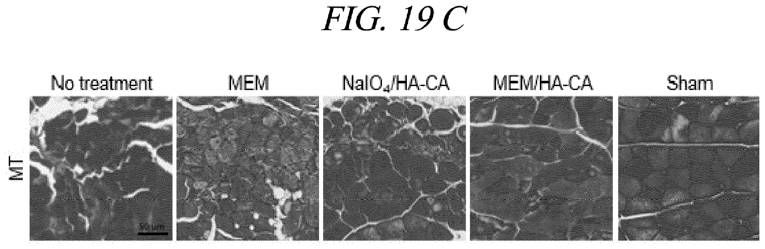

When the degree of fibrosis was analyzed by Masson's trichrome (MT) staining, it was confirmed that fibrotic collagen deposition occurred in all the groups compared with the sham (normal) group. Particularly, low collagen deposition occurred in the MEM/HA-CA group at a level most similar to that of the sham group, which confirms that scar tissue formation caused by tissue damage was the least in the MEM/HA-CA group (**FIGS. 19C** to **19D**).

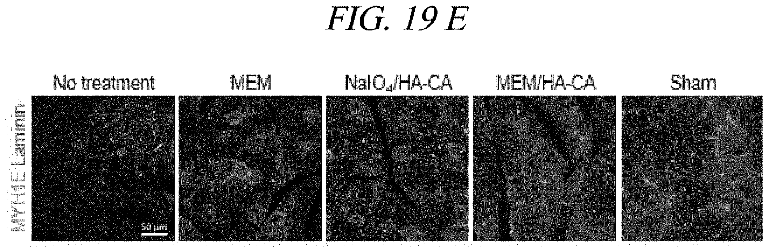

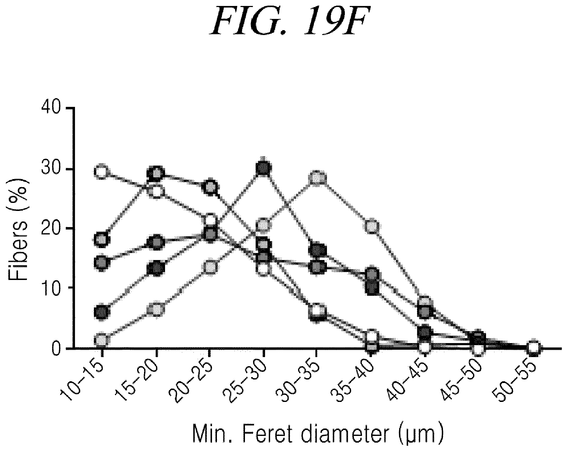

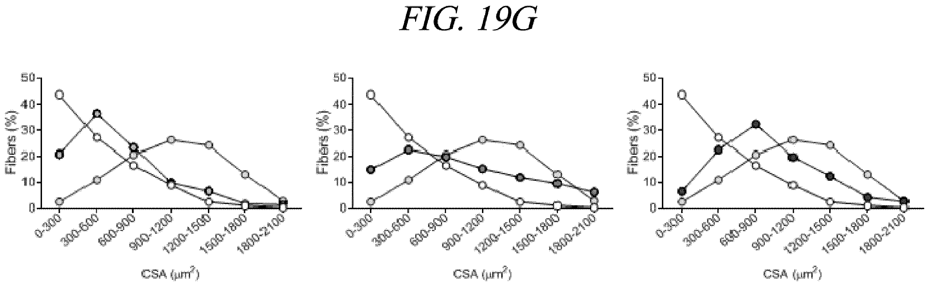

**As** a result of staining muscle-specific markers myosin heavy chain 1E (MYH1E) and laminin, MYH1E-positive myofiber was observed in all the groups except the no treatment group, and the MEM/HA-CA exhibited correct laminin deposition and a similar expression level of MYH1E to that of the shame group (**FIG. 19E**). When analyzing the minimum Feret diameter (**FIG. 19F**) and the cross-sectional area (CSA) (**FIG. 19G**) of myofibers, the myofiber size distribution in the MEM/HA-CA group was observed to be most similar to that of the sham group, which confirms that muscle tissue regeneration was successful.

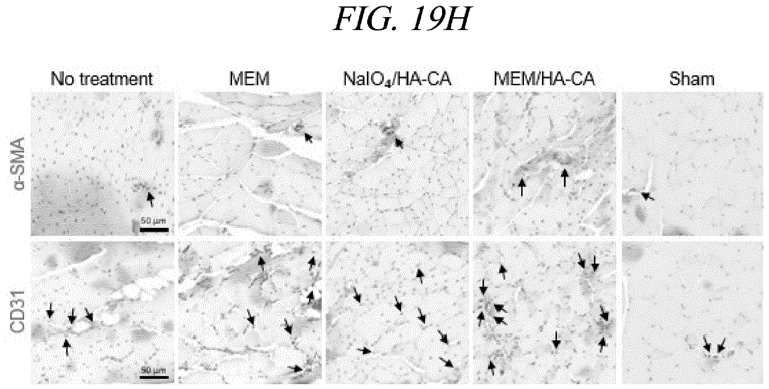

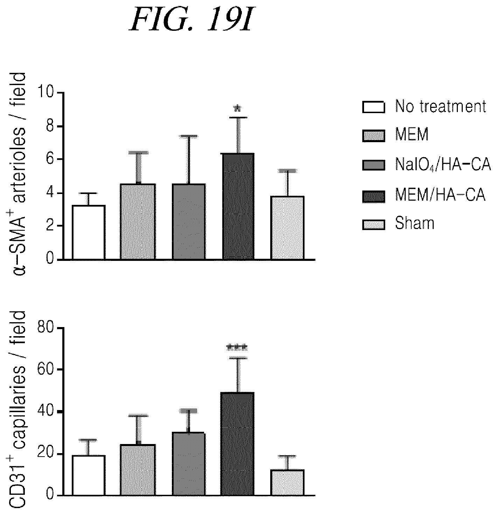

The regeneration and functional performance of the muscle can be maximized only when oxygen and nutrients are smoothly supplied through blood vessels in the muscle tissue. As a result of staining vascularization-related markers, α-smooth muscle actin (α-SMA) and CD31 (**FIG. 19H**), the number of α-SMA-positive arterioles and the number of CD31-positive microvessels were significantly increased in the MEM/HA-CA compared with the no treatment group (**FIG. 19I**).

Accordingly, it was confirmed that the MEM/HA-CA hydrogel patch can induce the regeneration of functionally improved muscle by inducing the regeneration of not only muscles but also surrounding tissues such as blood vessels.

### Example 2: Analysis of phenol group-functionalized hydrogel patch for tissue-derived extracellular matrix (ECM) delivery (2)

### 2-1. Fabrication of phenol group (gallol group)-functionalized hydrogel patch (MEM/HA-PG) for muscle extracellular matrix (MEM) delivery

A pyrogallol-conjugated adhesive hyaluronic acid (HA-PG) derivative functionalized with a gallol group, which is another phenol group, was synthesized to fabricate an MEM/HA-PG hydrogel patch loaded with a muscle extracellular matrix (MEM).

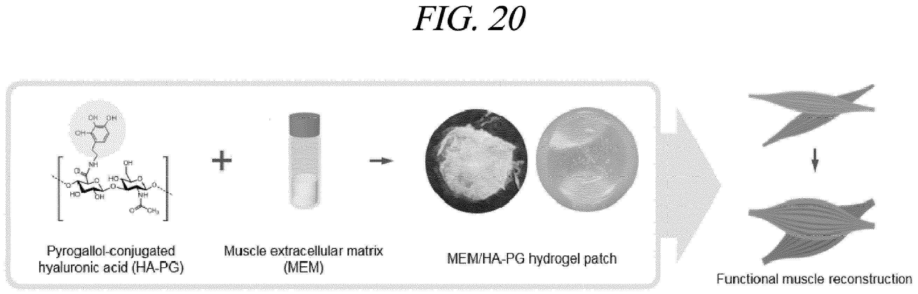

**As** for the fabricated MEM/HA-PG patch like the MEM/HA-CA patch, cross-linking and hydrogel formation occur via reaction between the MEM and the gallol group. Thus, the MEM/HA-PG patch can induce muscle tissue regeneration through efficient delivery of the MEM (**FIG. 20**).

**A** decellularized muscle extracellular matrix used herein is the same as described above.

### 2-2. Analysis on mechanical properties of MEM/HA-PG hydrogel patch

The HA-PG derivative is expected to induce rapid cross-linking due to the excellent self-oxidation ability of the gallol group and have high reactivity with various components present in MEM, which was checked.

Specifically, the MEM was added into a 0.5% HA-PG derivative (DS: 7%) solution to make final MEM concentrations of 0 µg/ml, 20 µg/ml, 60 µg/ml, 100 µg/ml and 140 µg/ml (groups 0, 20, 60, 100 and 140, respectively) and hydrogels were formed under various concentration conditions and the mechanical properties thereof were compared.

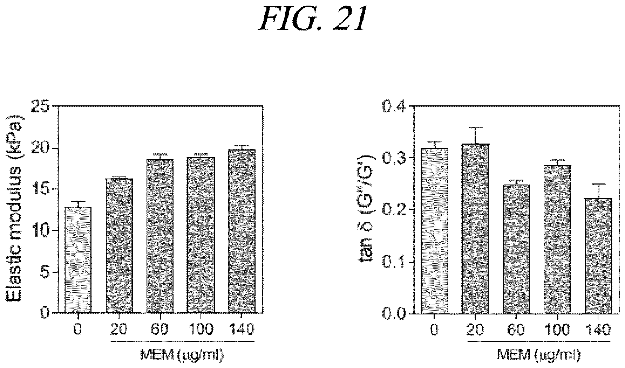

As the concentration of the MEM increased, the elastic modulus value increased and the tan delta value decreased (**FIG. 21**), which shows that the MEM component can induce enhancement of the physical properties of the HA-PG hydrogel patch.

### 2-3. Check of biocompatibility of MEM/HA-PG hydrogel patch

C2C12 (mouse myoblast) cells were seeded in the MEM/HA-PG hydrogel patches formed under various MEM concentration conditions and the status of the cells was observed during three-dimensional incubation for 7 days, and the cell viability was analyzed by performing live/dead staining.

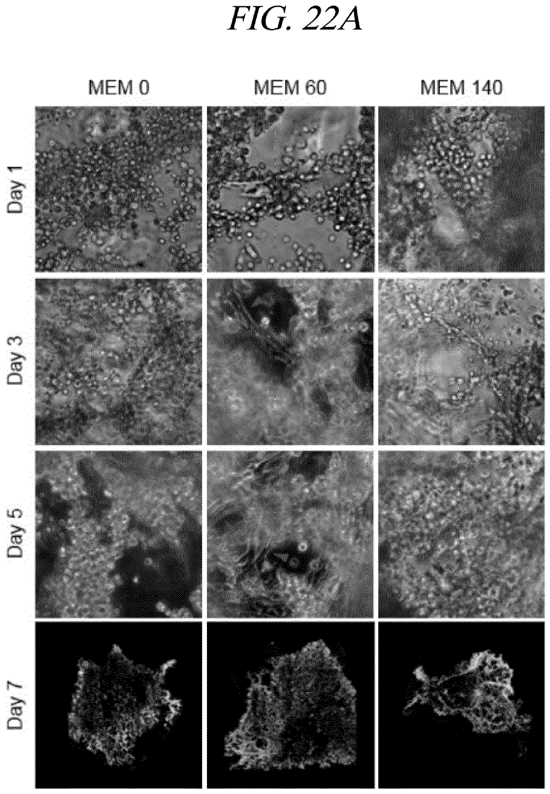

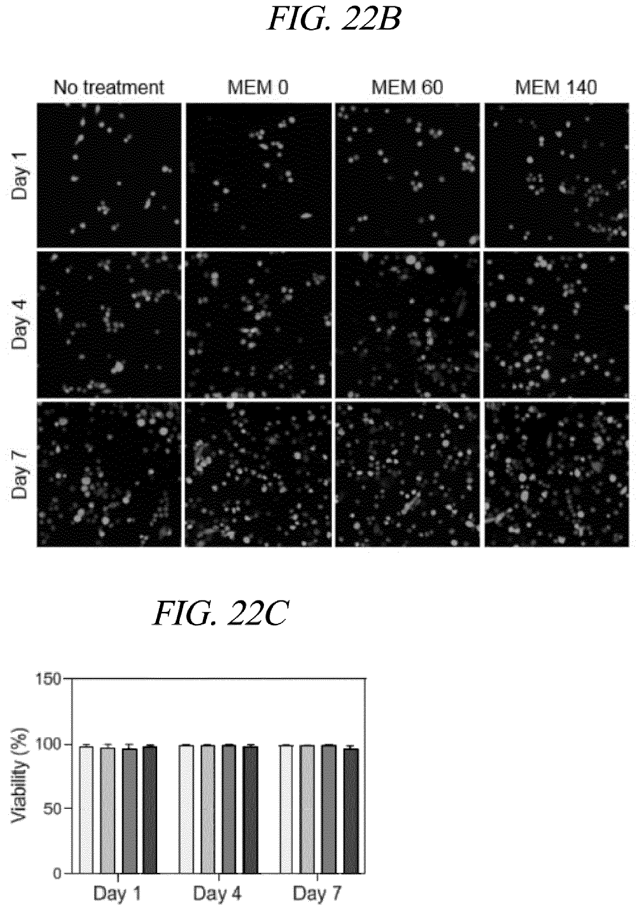

In the HA-PG hydrogel patch added with 60 µg/ml MEM (MEM 60, 0.5% HA-PG), the muscle tube structure was generated (red arrows) as incubation time passed and cells were grown well compared with the other groups. Also, it was confirmed that the cell viability was higher in the groups added with the MEM than in the group without the MEM (**FIG. 22A**).

*MEM 0: HA-PG patch without MEM d

*MEM 140: HA-PG patch added with 140 µg/ml MEM

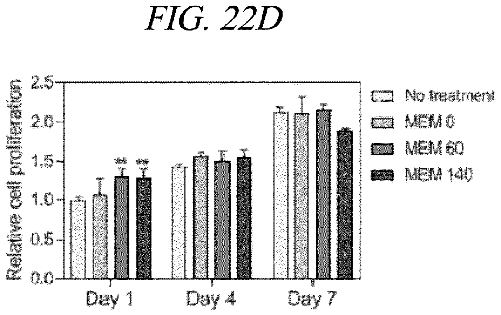

In addition, the MEM/HA-PG hydrogel patches of the same conditions were incubated under physiological conditions (37°C, muscle stem cell culture medium) for 24 hours to induce release of the loaded MEM and then, the culture medium was collected and used for treating mouse-derived muscle stem cells. Then, the cell viability (**FIGS. 22B** to **22C**) and the degree of proliferation (**FIG. 22D**) were evaluated. There was no significant difference in viability until day 7 of incubation. However, as a result of analyzing the proliferation, significant cell proliferation was observed in the group treated with the culture medium collected from the MEM-loaded HA-PG hydrogel patch after 1 day of incubation. Thus, it was found that the MEM loaded in the HA-PG hydrogel patch is involved in initial cell proliferation. Meanwhile, on day 7 of incubation, there was no difference or a slight decrease in the degree of proliferation between the group treated with the culture medium collected from the MEM-loaded HA-PG hydrogel patch and the group without any treatment (using a conventional muscle stem cell culture medium-No treatment group). Accordingly, it seems that as the amount of the released MEM increased, differentiation was further induced and cell proliferation was relatively decreased.

* The cell viability was analyzed by live/dead assay, and the cell proliferation was identified by MTT assay.

This shows that the MEM-loaded HA-PG hydrogel patch has excellent biocompatibility and can promote the proliferation and differentiation of muscle cells.

The present disclosure has been described with reference to the preferred exemplary embodiments thereof. It can be understood by a person with ordinary skill in the art that the present disclosure can be implemented as being modified and changed within the scope departing from the spirit and the scope of the present disclosure. Accordingly, the above-described exemplary embodiments should be considered in descriptive sense only and not for purposes of limitation. Also, the technical scope of the present disclosure is defined not by the detailed description of the invention but by the appended claims, and all differences within the scope will be construed as being comprised in the present disclosure.

## Claims

1. An extracellular matrix hydrogel patch, comprising:
a hydrogel patch containing a biocompatible polymer functionalized with a phenol group; and
an extracellular matrix loaded in the hydrogel patch.

2. The extracellular matrix hydrogel patch of Claim 1,
wherein the phenol group is a catechol group derived from a catechol-based compound selected from the group consisting of catechol, 4-tert-butylcatechol (TBC), urushiol, alizarin, dopamine, dopamine hydrochloride, 3,4-dihydroxyphenylalanine (DOPA), caffeic acid, norepinephrine, epinephrine, 3,4-dihydroxyphenylacetic acid (DOPAC), isoprenaline, isoproterenol and 3,4-dihydroxybenzoic acid; or a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

3. The extracellular matrix hydrogel patch of Claim 1,
wherein the biocompatible polymer is selected from the group consisting of hyaluronic acid, heparin, cellulose, dextran, alginate, chitosan, chitin, collagen, gelatin, chondroitin sulfate, pectin, keratin and fibrin.

4. The extracellular matrix hydrogel patch of Claim 1,
wherein the extracellular matrix hydrogel patch has
i) a thickness of from 0.05 mm to 10.0 mm,
ii) a storage modulus G' of from 1×10² Pa to 1×10⁶ Pa and tanδ of from 0.2 to 0.5 in a frequency range of from 0.1 Hz to 10 Hz,
iii) a friction factor of from 0.2 to 0.4 as measured at a speed of 0.01 m/s under a normal force of 5 N, and
iv) an adhesive strength of from 1 N to 10 N.

5. The extracellular matrix hydrogel patch of Claim 1,
wherein the extracellular matrix serves as a cross-linking agent via interaction with the phenol group.

6. The extracellular matrix hydrogel patch of Claim 1,
wherein the extracellular matrix is derived from any one or more tissues selected from the group consisting of muscle, brain, spinal cord, tongue, airway, skin, lymph, lung, heart, liver, stomach, kidney, spleen, pancreas, intestine, adrenal gland, fat, uterus, thymus, esophagus, salivary gland, bone, bladder, blood vessel, tendon, thyroid and gum.
